(19) Europäisches Patentamt
European Patent Office
Office européen des brevets

(11) EP 2 526 965 A1

(12) **DEMANDE DE BREVET EUROPEEN**

(43) Date de publication:
**28.11.2012 Bulletin 2012/48**

(51) Int Cl.:
***A61K 39/12*** *(2006.01)* ***A61P 31/14*** *(2006.01)*

(21) Numéro de dépôt: **11192672.1**

(22) Date de dépôt: **02.10.2007**

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **04.10.2006 FR 0608660**

(62) Numéro(s) de document de la (des) demande(s) initiale(s) en application de l'article 76 CBE:
**07858487.7 / 2 077 857**

(71) Demandeur: **Sanofi Pasteur**
**69367 Lyon Cedex 07 (FR)**

(72) Inventeurs:
- **Guy, Bruno**
  **69002 Lyon (FR)**
- **Forrat, Rémi**
  **F-69780 Serezin du Rhône (FR)**
- **Lang, Jean**
  **F-69780 Mions (FR)**
- **Barban, Véronique**
  **F69290 Craponne (FR)**

Remarques:
Cette demande a été déposée le 08-12-2011 comme demande divisionnaire de la demande mentionnée sous le code INID 62.

# (54) Méthode d'immunisation contre les 4 sérotypes de la Dengue

(57) L'invention concerne une méthode pour induire une protection contre les 4 sérotypes de la dengue chez un patient, comprenant :
(a) une première série d'administrations (i) d'une dose d'un virus vaccinal de la dengue d'un premier sérotype et d'une dose d'un virus vaccinal de la dengue d'un deuxième sérotype, et (ii) d'une dose d'un virus vaccinal de la dengue d'un troisième sérotype et d'une dose d'un virus vaccinal de la dengue d'un quatrième sérotype et
(b) une deuxième série d'administrations des doses (i) et (ii),

dans laquelle les doses (i) et (ii) sont administrées simultanément à des sites anatomiques distincts, et
dans laquelle la deuxième série est mise en oeuvre au moins 30 jours à au plus 12 mois après la première série.

EP 2 526 965 A1

## Description

L'invention a pour objet une méthode permettant d'induire une protection contre les 4 sérotypes de la dengue chez un patient, comprenant

(a) une première série d'administrations (i) d'une dose d'un virus vaccinal de la dengue d'un premier sérotype et d'une dose d'un virus vaccinal de la dengue d'un deuxième sérotype, et (ii) d'une dose d'un virus vaccinal de la dengue d'un troisième sérotype et d'une dose d'un virus vaccinal de la dengue d'un quatrième sérotype et
(b) une deuxième série d'administrations des doses (i) et (ii),

dans laquelle les doses (i) et (ii) sont administrées simultanément à des sites anatomiques distincts, et
dans laquelle la deuxième série (b) est mise en oeuvre au moins 30 jours à au plus 12 mois après la première série (a).

Les maladies dengue sont causées par quatre virus du genre flavivirus de type sérologique proches mais distincts d'un point de vue antigénique (Gübler et al., 1988 In: Epidemiology of arthropod-borne viral disease. Monath TPM, editor, Boca Raton (FL): CRC Press: 223-60 ; Kautner et al., 1997, J. of Pediatrics, 131:516-524; Rigau-Pérez et al., 1998, Lancet; 352: 971-977 ; Vaughn et al., 1997, J Infect Dis; 176: 322-30). L'infection avec un sérotype de la dengue peut produire un spectre de maladie clinique allant d'un syndrome viral non spécifique jusqu'à une maladie sévère hémorragique fatale. La période d'incubation de la fièvre dengue après piqûre du moustique est d'environ 4 jours (allant de 3 à 14 jours). La fièvre dengue est caractérisée par une fièvre biphasique, des maux de tête, des douleurs dans différentes parties du corps, une prostration, des éruptions, une lymphadénopathie et une leucopénie (Kautner et al., 1997, J. of Pediatrics, 131:516-524 ; Rigau-Pérez et al., 1998, Lancet; 352: 971-977). La période virémique est la même que la période fébrile (Vaughn et al., 1997, J. Infect. Dis.; 176: 322-30). La guérison de la fièvre dengue est acquise au bout de 7 à 10 jours, mais une asthénie prolongée est usuelle. Des baisses de la numération des leucocytes et des plaquettes sont fréquentes.

La dengue hémorragique est une maladie fébrile sévère caractérisée par des anomalies de l'homéostasie et une augmentation de la perméabilité vasculaire qui peut conduire à une hypovolémie et à une hypotension (dengue avec syndrome de choc) souvent compliquée par des hémorragies internes sévères. Le taux de mortalité de la dengue hémorragique peut atteindre jusqu'à 10 % sans thérapie, mais est de 1 % dans la plupart des centres ayant une expérience thérapeutique (WHO technical Guide, 1986. Dengue haemorrhagic fever: diagnosis, treatment and control, p1-2. World Health Organization, Geneva, Switzerland).

Le diagnostic de routine en laboratoire de la dengue est basé sur l'isolation du virus et/ou la détection d'anticorps spécifiques du virus de la dengue.

La dengue est la deuxième maladie infectieuse tropicale la plus importante après la malaria, plus de la moitié de la population mondiale vivant dans des zones à risque de transmission épidémique. Chaque année, les cas de dengue sont estimés à 50-100 millions, les cas de patients hospitalisés pour une dengue hémorragique à 500 000, et le nombre de morts à 25 000. La dengue est endémique en Asie, dans le Pacifique, en Afrique, en Amérique latine et dans les Caraïbes. Plus de 100 pays tropicaux sont endémiques pour les infections par le virus de la dengue et la dengue hémorragique a été documentée dans 60 de ces pays (Gubler, 2002, TRENDS in Microbiology. 10:100-103 ; Monath, 1994, Proc. Natl. Acad. Sci.; 91: 2395-2400). Un certain nombre de facteurs bien décrits semblent être impliqués dans la dengue : la croissance de la population ; une urbanisation non planifiée et non contrôlée, en particulier en association avec la pauvreté ; une augmentation des voyages aériens ; le manque de contrôle effectif des moustiques et la détérioration des infrastructures sanitaires et de santé publique (Gubler, 2002, TRENDS in Microbiology. 10: 100-103). Les voyageurs et expatriés sont de plus en plus alertés contre la dengue (Shirtcliffe et al., 1998, J. Roy. Coll. Phys. Lond.; 32: 235-237). La dengue a constitué une des principales causes de maladies fébriles au sein les troupes américaines au cours de déploiements dans des zones tropicales endémiques pour la dengue (DeFraites et al., 1994, MMWR 1994; 43: 845-848).

Les virus sont maintenus dans un cycle qui implique les humains et *Aedes aegypti,* u n moustique domestique piquant le jour, qui préfère s'alimenter sur l'homme. L'infection chez l'homme est initiée par l'injection du virus au cours du repas de sang d'un moustique *A*edes *aegypti* infecté. Le virus salivaire est déposé principalement dans les tissus extravasculaires. La première catégorie de cellules infectées après inoculation sont les cellules dendritiques, qui migrent ensuite vers les ganglions lymphatiques (Wu et al., 2000, Nature Med.; 7:816-820). Après une réplication initiale dans la peau et dans les ganglions lymphatiques, le virus apparaît dans le sang au cours de la phase fébrile aiguë, généralement pendant 3 à 5 jours.

Les monocytes et les macrophages sont, avec les cellules dendritiques, parmi les premières cibles du virus de la dengue. La protection contre une réinfection homotypique est complète et dure probablement toute la vie, mais une protection croisée entre les différents types de dengue dure moins de quelques semaines à quelques mois (Sabin, 1952, Am. J. Trop. Med. Hyg.; 1: 30-50). En conséquence, un sujet peut subir une infection avec un sérotype différent. Une seconde infection par la dengue est en théorie un facteur de risque de développer une maladie dengue sévère.

Cependant, la dengue hémorragique est multifactorielle : ces facteurs incluent la souche du virus impliqué, ainsi que l'âge, le statut immun et la prédisposition génétique du patient. Deux facteurs jouent un rôle majeur dans la survenue de la dengue hémorragique: une réplication virale rapide avec une virémie élevée (la sévérité de la maladie étant associée au niveau de la virémie ; Vaughn et al., 2000, J. Inf. Dis.; 181: 2-9) et une réponse inflammatoire importante avec la libération de niveaux élevés de médiateurs inflammatoires (Rothman and Ennis, 1999, Virology; 257: 1-6). Il n'y a aucun traitement spécifique contre la dengue. Le traitement de la fièvre dengue est symptomatique avec un alitement, un contrôle de la fièvre et de la douleur avec des antipyrétiques et des analgésiques, et une prise de boisson adéquate. Le traitement de la dengue hémorragique nécessite l'équilibrage des pertes de liquide, le remplacement des facteurs de coagulation et la perfusion d'héparine.

Les mesures de prévention reposent actuellement sur le contrôle du vecteur et des mesures de protection personnelle qui sont difficiles à mettre en oeuvre et coûteuses. Aucun vaccin contre la dengue n'est approuvé pour le moment. Etant donné que les quatre sérotypes de la dengue sont en circulation dans le monde et comme ils ont été rapportés comme étant impliqués dans des cas de fièvre hémorragique dengue, la vaccination devrait idéalement conférer une protection contre les quatre sérotypes du virus de la dengue.

L'utilisation de sites anatomiques différents pour l'administration de virus de la dengue a déjà été décrite dans la littérature.

Ainsi, Halstead et al. (1973, Am. J. Trop. Med. Hyg., 22 :375-381) ont montré qu'une administration de virus dengue sauvages effectuée en 2 ou 4 sites anatomiques séparés pour les 4 sérotypes différents induisait une protection contre une infection ultérieure. Cependant, les auteurs n'ont observé aucune supériorité de ce type d'immunisation séparée par rapport à une immunisation conduite en un seul site.

Les formes virales atténuées des virus dengue conduisent à des interférences chez l'homme lorsqu'elles sont administrées sous forme de vaccin tétravalent. Ce phénomène a en particulier été décrit dans les publications suivantes : Gubler D.J. Clin. Microbiol. Rev. 1998 ; 11 (3): 480-96 ; Rothman A. L. et al Vaccine 2001 ; 19 : 4694-9.

Zhou H et Deem MW. (Vaccine. 2006 Mar 24;24(14):2451-9) ont développé un modèle mathématique basé uniquement sur l'utilisation des épitopes CD8 et visant à simuler les interférences entre les épitopes CD8 des 4 sérotypes de la dengue. Selon ce modèle théorique, la meilleure façon d'éviter les interférences serait de faire une primo immunisation utilisant un épitope CD8 non dominant suivie d'un rappel par une administration à des sites anatomiques différents des mêmes épitopes CD8 de chacun des 4 sérotypes.

Il existe donc un besoin d'une méthode permettant de réduire les interférences entre les différents sérotypes et permettant d'induire des anticorps neutralisant contre les 4 sérotypes de la dengue.

Les inventeurs ont mis en évidence qu'il est possible de générer une réponse immune homologue comprenant des anticorps neutralisant les 4 sérotypes lorsque ces derniers sont administrés simultanément deux par deux en des sites anatomiques distincts lors d'une première série d'administrations puis d'une deuxième série d'administrations mise en oeuvre 30 jours à 12 mois après la première administration des 4 sérotypes.

Les inventeurs ont en particulier montré qu'une immunisation bivalente DEN-1,2 concomitante à une immunisation bivalente DEN-3,4 réalisée au niveau de deux sites anatomiques distincts et suivie d'un rappel des mêmes doses vaccinales dans les mêmes conditions induit des réponses contre les quatre sérotypes chez tous les singes immunisés, à l'exception d'un sérotype chez un animal. A l'inverse, une immunisation tétravalente conduite en un seul site n'a permis d'induire une réponse satisfaisante que contre deux sérotypes sur 4.

La réponse immune générée par la méthode selon la présente invention est donc plus importante quantitativement et qualitativement (couvre tous les sérotypes).

Selon un premier objet, la présente invention concerne donc une méthode permettant d'induire une protection homologue contre les 4 sérotypes de la dengue chez un patient, comprenant

(a) une première série d'administrations (i) d'une dose d'un virus vaccinal de la dengue d'un premier sérotype et d'une dose d'un virus vaccinal de la dengue d'un deuxième sérotype et (ii) d'une dose d'un virus vaccinal de la dengue d'un troisième sérotype et d'une dose d'un virus vaccinal de la dengue d'un quatrième sérotype et
(b) une deuxième série d'administrations des doses (i) et (ii),

dans laquelle les doses (i) et (ii) sont administrées simultanément à des sites anatomiques distincts, et
dans laquelle la deuxième série est mise en oeuvre au moins 30 jours à au plus 12 mois après la première série.

Selon un autre mode de réalisation de la méthode selon l'invention, les virus vaccinaux de la dengue (i) sont administrés sous forme d'une dose vaccinale bivalente unique.

Selon un autre mode de réalisation de la méthode selon l'invention, les virus vaccinaux de la dengue (ii) sont administrés sous forme d'une dose vaccinale bivalente unique.

Selon un mode de réalisation particulier de la méthode d'immunisation selon l'invention, ledit virus vaccinal de la dengue de sérotype 1 est sélectionné dans le groupe constitué de la souche VDV1 et d'un ChimeriVax™ DEN-1.

Selon un autre mode de réalisation particulier de la méthode selon l'invention, ledit virus vaccinal de la dengue de sérotype 2 est sélectionné dans le groupe constitué de la souche VDV2 et d'un ChimeriVax™ DEN-2.

Selon un autre mode de réalisation particulier de la méthode selon l'invention, ledit virus vaccinal de la dengue de sérotype 1 est la souche VDV1 et ledit virus vaccinal de la dengue de sérotype 2 est la souche VDV2.

Selon un autre mode de réalisation particulier de la méthode selon l'invention, ledit virus vaccinal de la dengue de sérotype 1 est un ChimeriVax™ DEN-1 et ledit virus vaccinal de la dengue de sérotype 2 est un ChimeriVax™ DEN-2.

Selon un autre mode de réalisation particulier de la méthode selon l'invention, ledit virus vaccinal de la dengue de sérotype 3 est un ChimeriVax™ DEN-3.

Selon un autre mode de réalisation particulier de la méthode selon l'invention, ledit virus vaccinal de la dengue de sérotype 4 est un ChimeriVax™ DEN-4.

Selon un autre mode de réalisation particulier de la méthode selon l'invention, les premier et deuxième sérotypes sont respectivement CYD DEN-1 et CYD DEN-2 et les troisième et quatrième sérotypes sont respectivement CYD DEN-3 et CYD DEN-4.

Selon un autre mode de réalisation particulier de la méthode selon l'invention, les premier et deuxième sérotypes sont respectivement CYD DEN-1 et CYD DEN-3 et les troisième et quatrième sérotypes sont respectivement CYD DEN-2 et CYD DEN-4.

Selon un autre mode de réalisation particulier de la méthode selon l'invention, la quantité de virus vaccinaux de la dengue de sérotypes 1, 2, 3 et 4 est comprise dans une gamme allant de $10^3$ à $10^6$ $DICC_{50}$.

Selon un autre mode de réalisation de la méthode selon l'invention, les virus vaccinaux utilisés dans la deuxième série d'administrations sont identiques à ceux utilisés dans la première série d'administration.

Selon un autre mode de réalisation de la méthode selon l'invention, la deuxième série d'administrations est mise en oeuvre 30 à 60 jours après la première série d'administrations.

La présente invention a également pour objet un kit d'immunisation contre le virus de la dengue comprenant un boitier contenant au moins les virus vaccinaux de la dengue des sérotypes 1, 2, 3 et 4

(a) sous la forme de compositions monovalentes contenues dans 4 récipients séparés, ou
(b) sous la forme de deux compositions bivalentes contenues dans 2 récipients séparés.

Selon un mode de réalisation, le kit selon l'invention comprend au moins:

(a) un premier récipient contenant un vaccin bivalent comprenant un ChimeriVax™ DEN-1 et un ChimeriVax™ DEN-2, et
(b) un deuxième récipient contenant un vaccin bivalent comprenant un ChimeriVax™ DEN-3 et un ChimeriVax™ DEN-4.

Selon un autre mode de réalisation, le kiit selon le kit selon l'invention comprend au moins:

(a) un premier récipient contenant un vaccin bivalent comprenant un ChimeriVax™ DEN-1 et un ChimeriVax™ DEN-3, et
(b) un deuxième récipient contenant un vaccin bivalent comprenant un ChimeriVax™ DEN-2 et un ChimeriVax™ DEN-4.

La présente invention a également pour objet un kit d'immunisation contre le virus de la dengue comprenant un boitier contenant au moins les virus vaccinaux de la dengue d'un premier et d'un deuxième sérotypes,

(a) sous la forme de 2 compositions monovalentes contenues dans 2 récipients séparés, ou
(b) sous la forme d'une composition bivalente contenue dans 1 seul récipient.

Selon un mode de réalisation, le kit comprend au moins :

(a) un récipient contenant un vaccin bivalent comprenant un ChimeriVax™ DEN-1 et un ChimeriVax™ DEN-3, ou
(b) un récipient contenant un vaccin bivalent comprenant un ChimeriVax™ DEN-2 et un ChimeriVax™ DEN-4, ou
(c) un récipient contenant un vaccin bivalent comprenant un ChimeriVax™ DEN-1 et un ChimeriVax™ DEN-2, ou
(d) un récipient contenant un vaccin bivalent comprenant un ChimeriVax™ DEN-3 et un ChimeriVax™ DEN-4.

La présente invention fournit également une composition ou un vaccin bivalent comprenant une quantité immunoefficace des virus vaccinaux de la dengue d'un premier et d'un deuxième sérotypes et un excipient pharmaceutiquement acceptable.

Selon un mode de réalisation particulier, la composition ou vaccin bivalent comprend les virus vaccinaux sélectionnés dans le groupe consistant en : ChimeriVax™ DEN-1 et ChimeriVax™ DEN-3 ; ou ChimeriVax™ DEN-2 et ChimeriVax™ DEN-4 ; ou ChimeriVax™ DEN-1 et un ChimeriVax™ DEN-2 ; ou ChimeriVax™ DEN-3 et ChimeriVax™ DEN-4.

L'invention va être décrite plus en détails dans la description qui suit.

*Définitions*

Dans le cadre de la présente invention, deux sites anatomiques sont « distincts », s'ils sont drainés par des ganglions lymphatiques différents. Par exemple, le bras droit et le bras gauche sont considérés comme des sites distincts. On peut également citer à titre d'exemples non limitatifs les sites distincts suivants: le bras droit/la cuisse droite ; le bras gauche /la cuisse gauche, le bras gauche/la cuisse droite.

On entend dans le cadre de la présente invention par « administrations simultanées » des administrations mises en oeuvre le même jour (i.e. au plus 24H). Des administrations simultanées sont avantageusement réalisées à au plus 1 heure d'intervalle l'une de l'autre, classiquement à 1-5 minutes l'une de l'autre.

Dans le cadre de présente invention, les doses (i) sont administrées au niveau d'un premier site anatomique, soit sous la forme de deux doses monovalentes, soit sous la forme d'une dose bivalente unique. Les doses (ii) sont quant à elles administrées simultanément au niveau d'un deuxième site anatomique, soit sous la forme de deux doses monovalentes, soit sous la forme d'une dose bivalente unique, les premier et deuxième sites étant des sites distincts tels que définis ci-dessus.

Les « virus de la dengue » ou « DEN » sont des virus à ARN simple-brin, positif, appartenant au genre Flavivirus de la famille des *flaviviridae.* L'ARN génomique contient une coiffe de type I à l'extrémité 5' mais est dépourvu de queue poly-A à l'extrémité 3'. L'organisation génomique est constituée des éléments suivants : région non codante (NCR) 5', protéines structurales (capside (C), pré-membrane/membrane (prM/M), enveloppe (E)) et protéines non structurales (NS1-NS2A-NS2B-NS3-NS4A-NS4B-NS5) et NCR 3'. L'ARN viral génomique est associé aux protéines de la capside pour former une nucléocapside. Comme pour les autres flavivirus, le génome viral DEN code une région codante ininterrompue qui est traduite en une polyprotéine unique.

Par « virus vaccinal de la dengue» on entend dans le cadre de la présente invention, toute forme virale du virus de la dengue capable d'induire une réponse immune homologue spécifique, de préférence toute forme virale du virus de la dengue utilisable dans le cadre d'un programme d'immunisation chez l'homme contre une infection par un virus de la dengue. Par virus vaccinaux de la dengue on entend donc les virus inactivés, les virus atténués, ou les protéines recombinantes telles que la protéine d'enveloppe du virus de la dengue.

Un virus vaccinal est considéré comme « inactivé » s'il ne se réplique plus sur des cellules permissives.

Un virus vaccinal est considéré comme « atténuée » si après croissance à 37°C ou 39°C sur cellule hépatique Huh-7, VERO et/ou C6/36, ledit virus vaccinal présente un titre maximal inférieur d'au moins 10 fois au titre maximal obtenu avec la souche parentale sauvage dans les mêmes conditions de culture et tel que mesuré en utilisant la même méthode de titrage. Un virus vaccinal qui présente une croissance diminuée sur au moins l'un des trois types cellulaires identifiés ci-dessus est donc considéré comme « atténué » dans le cadre de la présente invention.

Un virus vaccinal utilisable chez l'homme présente un rapport bénéfices/risques positif, ledit rapport permet généralement de satisfaire aux exigences réglementaires pour l'obtention d'une autorisation de mise sur le marché. Un virus vaccinal de la dengue utilisé dans le cadre de la présente invention est de préférence un virus atténué de telle sorte qu'il n'induise pas la maladie chez l'homme. Avantageusement, ledit virus vaccinal ne conduit qu'à des effets secondaires au plus d'intensité modérée (i.e. moyenne à faible, voire nulle) chez la majorité des sujets vaccinés tout en conservant sa capacité à induire une réponse anticorps neutralisante.

On peut citer à titre d'exemples non limitatifs, de virus vaccinal de la dengue utilisable dans le cadre de la présente invention : les virus vaccinaux inactivés, les virus vaccinaux atténués tels que les souches atténuées VDV-1, VDV-2, les souches décrites par exemple dans les demandes : WO02/66621, WO0057904, WO0057908, WO0057909; WO0057910, WO02/0950075 et WO02/102828, ou les chimères. Les virus chimères ont la particularité de présenter les caractéristiques des virus atténués tels que définis ci-dessus. Tout virus chimères exprimant la protéine d'enveloppe d'un virus dengue et induisant une réponse immune comprenant des anticorps neutralisant le sérotype dont est issue la protéine d'enveloppe peut donc être utilisé dans le cadre de la présente invention. On peut citer à titre d'exemples non limitatifs : les ChimeriVax ™dengue tels que décrits par exemple dans la demande de brevet WO 98/37911, les chimères dengue/dengue tels que décrits par exemple dans les demandes de brevets WO9640933 et WO0160847.Le virus vaccinal de la dengue de sérotype 1 peut être par exemple la souche vaccinale VDV1 ou un ChimeriVax™ DEN-1, en particulier un virus YF17D/DEN-1, ou encore une souche DEN-1 16007/PDK13. Le virus vaccinal de la dengue de sérotype 2 peut être par exemple la souche vaccinale VDV2 ou un ChimeriVax™ DEN-2, en particulier un virus YF17D/DEN-2, ou encore une souche DEN-2 16681/PDK53. Le virus vaccinal de la dengue de sérotype 3 peut être un ChimeriVax™ DEN-3, en particulier un virus YF17D/DEN-3. Le virus vaccinal de la dengue de sérotype 4 peut être un ChimeriVax™ DEN-4, en particulier un virus YF17D/DEN-4.. Cette souche a été décrite dans la demande de brevet EP1159968 au nom de Mahidol University et a été déposée auprès de la Collection Nationale de Cultures de Microorganismes (CNCM) sous le numéro I-2483.

« VDV » ou « vaccin dengue Vero» désigne une souche virale dengue vivante atténuée adaptée sur cellule Vero et capable d'induire une réponse humorale spécifique, y compris l'induction d'anticorps neutralisants, chez les primates et en particulier chez l'homme.

« VDV-1 » est une souche obtenue à partir d'une souche sauvage DEN-1 16007 ayant subi 11 passages sur cellules PDK (DEN-1 16007/PDK11) qui a ensuite été amplifiée sur cellules Vero à 32°C, et dont l'ARN a été purifié et transfecté dans des cellules Vero. La souche VDV-1 présente 14 mutations supplémentaires par rapport à la souche vaccinale DEN-1 16007/PDK13 (13 passages sur cellules PDK-Primary Dog Kidney). La souche DEN-1 16007/PDK13, aussi appelée « LAV1 », a été décrite dans la demande de brevet EP1159968 au nom de Mahidol University et a été déposée auprès de la Collection Nationale de Cultures de Microorganismes (CNCM) sous le numéro I-2480. La séquence complète de la souche VDV-1 est donnée à la séquence SEQ ID NO :1. Ladite souche peut être facilement reproduite à partir de ladite séquence. Un procédé de préparation et la caractérisation de la souche VDV-1 ont été décrits dans la demande de brevet internationale déposée aux noms de Sanofi-Pasteur et du Center for Disease Control and Prevention sous le numéro PCT/IB 2006/001313.

« VDV-2 » est une souche obtenue à partir d'une souche sauvage DEN-2 16681 ayant subi 50 passages sur cellules PDK (DEN-2 16681/PDK50), purifiée par plaque et dont l'ARN a été extrait et purifié avant d'être transfecté dans des cellules Vero. La souche VDV-2 a ensuite été obtenue par purification par plaque et amplification sur cellules Vero. La souche VDV-2 présente 10 mutations supplémentaires par rapport à la souche vaccinale DEN-2 16681/PDK53 (53 passages sur cellules PDK), dont 4 mutations silencieuses. La souche DEN-2 16681/PDK53, aussi appelée « LAV2 », a été décrite dans la demande de brevet EP1159968 au nom de Mahidol University et a été déposée auprès de la Collection Nationale de Cultures de Microorganismes (CNCM) sous le numéro I-2481. La séquence complète de la souche VDV-2 est montrée dans la séquence SEQ ID NO :2. La souche VDV-2 peut être facilement reproduite à partir de ladite séquence. Un procédé de préparation et la caractérisation de la souche VDV-2 a été décrit dans la demande de brevet internationale déposée aux noms de Sanofi-Pasteur et du Center for Disease Control and Prevention sous le numéro PCT/IB 2006/001513.

Les souches VDV 1 et 2 sont préparées par amplification sur cellules Vero. Les virus produits sont récoltés et clarifiés des débris cellulaires par filtration. L'ADN est digéré par traitement enzymatique. Les impuretés sont éliminées par ultrafiltration. Les titres infectieux peuvent être augmentés par une méthode de concentration. Après ajout d'un stabilisant, les souches sont stockées sous forme lyophilisée ou congelée avant utilisation puis reconstituées extemporanément.

Par « ChimeriVax™ dengue» ou « CYD » on désigne un virus de la fièvre jaune (YF) chimère qui comprend le squelette d'un virus YF dans lequel les séquences codant pour les protéines de pré-membrane et d'enveloppe ont été remplacée par celles d'un virus DEN. On appelle ainsi « CYD-1 ou CYD DEN1 » un virus YF chimère contenant les séquences prM et E d'une souche dengue sérotype 1 (DEN-1). On appelle « CYD-2 ou CYD DEN2» un virus YF chimère contenant les séquences prM et E d'une souche DEN-2. On appelle « CYD-3 ou CYD DEN3» un virus YF chimère contenant les séquences prM et E d'une souche DEN-3. On appelle «CYD-4 ou CYD DEN4» un virus YF chimère contenant les séquences prM et E d'une souche DEN-4. La préparation de ces ChimeriVax™ dengue a été décrite en détails dans les demandes de brevet internationales WO 98/37911 et WO 03/101397 auxquelles on peut se reporter pour un descriptif précis de leur procédé de préparation. Les chimères décrits dans les exemples ont été générés par utilisation des séquences prM et E issues des souches DEN 1 PU0359 (TYP1140), DEN2 PU0218, DEN3 PaH881/88 et DEN 4 1288 (TVP 980). Toute souche du virus de la dengue pourrait être utilisée dans le cadre de la présente invention pour la construction des chimères.

De préférence, le virus YF chimère comprend le squelette d'une souche de la fièvre jaune atténuée YF17D (Theiler M, and Smith HH (1937) J Exp. Med 65, p767-786.) (virus YF17D/DEN-1, YF17D/DEN-2, YF17D/DEN-3, YF17D/DEN-4). Des exemples de souches YF17D susceptibles d'être utilisées incluent YF17D204 (YF-Vax®, Sanofi-Pasteur, Swifwater, PA, USA; Stamaril®, Sanofi-Pasteur, Marcy l'Etoile, France ; ARILVAX™, Chiron, Speke, Liverpool, UK ; FLAVIMUN®, Berna Biotech, Bern, Switzerland ; YF17D-204 France (X15067,X15062) ; YF17D-204,234 US (Rice et al., 1985, Science, 229:726-733), ou encore des souches apparentées YF17DD (Genbank numéro d'accès U17066), YF17D-213 (Genbank numéro d'accès U17067) et les souches YF17DD décrites par Galler et al. (1998, Vaccines 16 (9/10) :1024-1028). Toute autre souche de virus de la fièvre jaune atténuée utilisable chez l'homme peut être utilisée dans le cadre de la présente invention pour la construction des chimères.

La présente invention a donc également pour objet une composition ou vaccin bivalent comprenant une quantité immunoefficace d'un virus vaccinal de la dengue d'un premier sérotype et d'un virus vaccinal de la dengue d'un deuxième sérotype et un excipient pharmaceutiquement acceptable.

Pour un descriptif des virus vaccinaux utilisables dans les vaccins selon l'invention, on peut se référer à la description qui en est donnée dans le cadre de la méthode d'immunisation selon l'invention.

Selon un mode de réalisation particulier, la composition ou vaccin bivalent selon l'invention comprend CYD DEN-1 et CYD DEN-2, ou CYD DEN-3 et CYD DEN-4, ou CYD DEN-1 et CYD DEN-3 ou CYD DEN-2 et CYD DEN-4, avantageusement les virus vaccinaux sont présents dans le vaccin à une quantité de $10^5$ $DICC_{50}$.

Chaque virus vaccinal dengue monovalent ChimeriVax™ (sérotypes 1, 2, 3 et 4) a été préparé par amplification de chaque sérotype sur cellules Vero. Plus spécifiquement, les quatre virus sont produits séparément sur des cellules Vero adhérentes en milieu sans sérum. La récolte virale, clarifiée des débris cellulaires par filtration, est alors concentrée et purifiée par ultrafiltration et chromatographie pour enlever l'ADN des cellules hôtes. Après ajout d'un stabilisant, les souches vaccinales sont stockées sous forme congelée ou lyophilisée avant utilisation puis reconstituées extemporanément. Le même procédé est appliqué pour les quatre chimères.

Une dose, une composition ou un vaccin est « monovalent » lorsqu'il contient outre un excipient pharmaceutiquement acceptable un seul sérotype de virus dengue. Une dose, une composition ou un vaccin est « bivalent » lorsqu'il contient deux sérotypes différents de virus dengue. Une dose, une composition ou un vaccin est « trivalent » lorsqu'il contient trois sérotypes différents de virus dengue. Une dose, une composition ou un vaccin est « tétravalent » lorsqu'il contient quatre sérotypes différents de virus dengue. Les compositions multivalentes sont obtenues par simple mélange des compositions monovalentes.

Par « patient », on désigne une personne (enfant ou adulte) susceptible d'être infectée par la dengue, en particulier une personne à risque d'infection comme par exemple une personne voyageant dans des régions où la dengue est présente ou un habitant de ces régions. Ce terme englobe donc les personnes naïves ainsi que les personnes non-naïves pour le virus de la dengue.

*Immunisation simultanée en des sites anatomiques distincts*

Les inventeurs ont montré en particulier que l'administration des 4 sérotypes sous la forme de deux administrations bivalentes simultanées à des sites anatomiques distincts suivies d'un rappel 30 jours à 12 mois après la première série d'administrations permet d'obtenir une protection homologue efficace contre les 4 sérotypes. La méthode selon la présente invention présente donc un intérêt tout particulier dans le cadre d'une stratégie d'immunisation contre la dengue.

Selon la présente invention, les 4 sérotypes de la dengue peuvent être administrés dans un ordre quelconque pour autant qu'ils soient administrés deux par deux (i.e. doses (i) et (ii) respectivement sous forme de deux doses monovalentes ou d'une dose bivalente unique) de façon simultanée en des sites distincts.

La méthode selon la présente invention peut donc être mise en oeuvre avec les modes de réalisation décrits ci-dessous :

- (i) : sérotypes 1 et 2 ; (ii) sérotypes 3 et 4 ; ou
- (i) : sérotypes 1 et 3 ; (ii) sérotypes 2 et 4 ; ou
- (i) : sérotypes 1 et 4 ; (ii) sérotypes 2 et 3 ; ou
- (i) : sérotypes 2 et 3 ; (ii) sérotypes 1 et 4 ; ou
- (i) : sérotypes 2 et 4 ; (ii) sérotypes 1 et 3 ; ou
- (i) : sérotypes 3 et 4 ; (ii) sérotypes 1 et 2.

De préférence, la méthode selon la présente invention comprend l'administration des virus vaccinaux de la dengue suivants : (i) : sérotypes 1 et 2 ; (ii) sérotypes 3 et 4 ou (i) sérotypes 1 et 3 ; (ii) sérotypes 2 et 4. Les doses (i) et (ii) se présentent avantageusement sous la forme de doses bivalentes.

Selon des modes de réalisation particuliers, la présente invention couvre donc les schémas suivants :

- (i) CYD DEN-1 et CYD DEN-2 ; (ii) CYD DEN-3 et CYD DEN-4
- (i) CYD DEN-1 et CYD DEN-3 ; (ii) CYD DEN-2 et CYD DEN-4
- (i) CYD DEN-1 et CYD DEN-4 ; (ii) CYD DEN-2 et CYD DEN-3
- (i) CYD DEN-2 et CYD DEN-3 ; (ii) CYD DEN-1 et CYD DEN-4
- (i) CYD DEN-2 et CYD DEN-4 ; (ii) CYD DEN-1 et CYD DEN-3
- (i) CYD DEN-3 et CYD DEN-4 ; (ii) CYD DEN-1 et CYD DEN-2
- (i) VDV-1 et CYD DEN-2 ; (ii) CYD DEN-3 et CYD DEN-4
- (i) VDV-1 et CYD DEN-3 ; (ii) CYD DEN-2 et CYD DEN-4
- (i) VDV-1 et CYD DEN-4 ; (ii) CYD DEN-2 et CYD DEN-3
- (i) CYD DEN-2 et CYD DEN-3 ; (ii) VDV-1 et CYD DEN-4
- (i) CYD DEN-2 et CYD DEN-4 ; (ii) VDV-1 et CYD DEN-3
- (i) CYD DEN-3 et CYD DEN-4 ; (ii) VDV-1 et CYD DEN-2
- (i) CYD DEN-1 et VDV-2 ; (ii) CYD DEN-3 et CYD DEN-4
- (i) CYD DEN-1 et CYD DEN-3 ; (ii) VDV-2 et CYD DEN-4

- (i) CYD DEN-1 et CYD DEN-4 ; (ii) VDV-2 et CYD DEN-3
- (i) VDV-2 et CYD DEN-3 ; (ii) CYD DEN-1 et CYD DEN-4
- (i) VDV-2 et CYD DEN-4 ; (ii) CYD DEN-1 et CYD DEN-3
- (i) CYD DEN-3 et CYD DEN-4 ; (ii) CYD DEN-1 et VDV-2
- (i) VDV-1 et VDV-2 ; (ii) CYD DEN-3 et CYD DEN-4
- (i) VDV-1 et CYD DEN-3 ; (ii) VDV-2 et CYD DEN-4
- (i) VDV-1 et CYD DEN-4 ; (ii) VDV-2 et CYD DEN-3
- (i) VDV-2 et CYD DEN-3 ; (ii) VDV-1 et CYD DEN-4 et
- (i) VDV-2 et CYD DEN-4 ; (ii) VDV-1 et CYD DEN-3.
- (i) CYD DEN-3 et CYD DEN-4; (ii) VDV-1 et VDV-2 ;

De préférence, la méthode d'immunisation selon l'invention comprend l'administration des virus vaccinaux de la dengue suivants (i) CYD DEN-1 et CYD DEN-2 ; (ii) CYD DEN-3 et CYD DEN-4 ; ou (i) CYD DEN-1 et CYD DEN-3 ; (ii) CYD DEN-2 et CYD DEN-4. Les doses (i) et (ii) se présentent avantageusement sous la forme de doses bivalentes.

La méthode d'immunisation selon la présente invention comprend une deuxième série d'administrations réalisée de 30 jours à 12 mois, avantageusement de 30 jours à 3 mois, de préférence 30 jours, 45 jours ou 60 jours après la première série des administrations (i et ii) qui comprend avantageusement l'administration des mêmes compositions que celles utilisées lors de la première série qui sont administrées avantageusement dans les mêmes conditions.

On entend par « dose de virus vaccinal » dans le cadre de la présente invention une composition comprenant une « quantité immunoefficace » du virus vaccinal de la dengue, c'est-à-dire une quantité suffisante de virus de la dengue pour induire une réponse anticorps neutralisante homologue, qui peut être mise en évidence par exemple par le test de séroneutralisation tel que décrit ci-dessous dans l'exemple 1. Un sérum est considéré comme positif pour la présence d'anticorps neutralisants lorsque le titre d'anticorps neutralisants ainsi déterminé est supérieur ou égal à 1 :10 (unité : 1/dilution).

Les quantités de souche vaccinales sont exprimées communément en termes d'unité formant des plages virales (PFU) ou de dose infectant 50% de la culture tissulaire ou encore de dose infectant 50% de la culture cellulaire ($DICC_{50}$). Par exemple, les compositions selon l'invention peuvent contenir de 10 à $10^6$ $DICC_{50}$, en particulier de $10^3$ à $10^5$ $DICC_{50}$ de virus vaccinal dengue de sérotype 1, 2, 3 ou 4 pour une composition monovalente ou bivalente. Ainsi dans les compositions ou utilisation selon l'invention, les doses de virus vaccinaux de la dengue de sérotype 1, 2, 3 et 4 sont préférentiellement comprises chacune dans une gamme allant de 10 à $10^6$ $DICC_{50}$, tels que 10, $10^1$,$10^2$,$10^3$,$10^4$, $10^5$ ou $10^6$ $DICC_{50}$ en particulier dans une gamme allant de $10^3$ à $10^5$ $DICC_{50}$, Les virus vaccinaux peuvent être utilisés à des doses identiques ou différentes, qui peuvent être ajustées en fonction de la nature du virus vaccinal utilisé et de l'intensité de la réponse immunitaire obtenue.

Selon un mode de réalisation particulier de la méthode selon la présente invention, les doses, monovalentes ou bivalentes, de virus vaccinaux comprennent respectivement $10^5$ $DICC_{50}$ de CYD DEN-1, de CYD DEN-2, de CYD DEN-3 et de CYD DEN-4.

La réponse anticorps neutralisante est avantageusement durable, c'est-à-dire qu'elle peut être détectée dans le sérum au moins 6 mois, après la deuxième série des administrations (i) et (ii).

La dose d'un virus vaccinal de la dengue d'un premier sérotype et la dose d'un virus vaccinal de la dengue d'un deuxième sérotype (i.e. dose(s) (i)) sont administrées simultanément sous forme de deux compositions monovalentes, ou avantageusement sous la forme d'une composition ou dose bivalente unique.

De la même manière, la dose d'un virus vaccinal de la dengue d'un troisième sérotype et la dose d'un virus vaccinal de la dengue d'un quatrième sérotype (i.e. dose(s) (ii)) sont administrées simultanément sous forme de deux compositions vaccinales monovalentes, ou avantageusement sous la forme d'une composition vaccinale bivalente unique.

Les virus vaccinaux sont administrés sous forme de compositions vaccinales ou doses de virus vaccinaux qui peuvent être préparées selon n'importe quelle méthode connue de l'homme du métier. Habituellement, les virus, généralement sous forme lyophilisée, sont mélangés avec un excipient pharmaceutiquement acceptable, tel que de l'eau ou une solution saline tamponnée au phosphate, des agents mouillants ou stabilisants. Par « excipient pharmaceutiquement acceptable », on entend tout solvant, milieu de dispersion, charge etc, qui ne produit pas de réaction secondaire, par exemple allergique, chez l'humain ou l'animal. L'excipient est sélectionné en fonction de la forme pharmaceutique choisie, de la méthode et de la voie d'administration. Des excipients appropriés, ainsi que les exigences en matière de formulation pharmaceutique, sont décrites dans « Remington : The Science & Practice of Pharmacy », qui représente un ouvrage de référence dans le domaine.

De préférence, les compositions vaccinales sont préparées sous forme injectable, et peuvent correspondre à des solutions liquides, des suspensions ou des émulsions. Les compositions peuvent en particulier comprendre une solution aqueuse tamponnée de manière à maintenir un pH compris entre environ 6 et 9 (comme déterminé avec un pH-mètre à température ambiante).

Bien qu'il ne soit pas nécessaire de rajouter un adjuvant, Les compositions peuvent néanmoins comprendre un tel composé, c'est-à-dire une substance qui augmente, stimule, ou renforce, la réponse immunitaire cellulaire ou humorale induite par la souche vaccinale administrée simultanément. L'homme de l'art est à même de sélectionner parmi les adjuvants classiquement utilisés dans le domaine vaccinal, un adjuvant qui puisse être approprié dans le cadre de la présente invention.

Les compositions vaccinales selon l'invention peuvent être administrées selon n'importe quelle voie utilisée habituellement en vaccination, par exemple la voie parentérale (notamment intradermique, sous-cutanée, ou intramusculaire), avantageusement par voie sous-cutanée. De préférence, les compositions vaccinales sont des compositions injectables administrées par voie sous-cutanée au niveau de la région du deltoïde gauche et du deltoïde droit.

Le volume de composition administré dépend de la voie d'administration. Pour des injections sous-cutanées, le volume est généralement compris entre 0,1 et 1,0 ml, de préférence environ 0,5 ml.

La période optimale pour l'administration de la totalité des sérotypes 1 à 4, est d'environ 1 à 3 mois avant l'exposition au virus de la dengue. Les vaccins peuvent être administrés en tant que traitement prophylactique d'une infection par un virus de la dengue chez les adultes et les enfants. Les populations ciblent incluent donc des personnes qui peuvent être naïves (i.e. non préalablement immunisés) ou non-naïves vis-à-vis du virus de la dengue.

Des administrations de rappel des virus vaccinaux de la dengue de sérotypes 1 à 4 peuvent en outre être mises en oeuvre par exemple entre 6 mois et 10 ans, par exemple 6 mois, 1 ans, 3 ans, 5 ans ou 10 ans après l'administration de la deuxième série d'administrations selon l'invention. Les administrations de rappel vont être mises en oeuvre avantageusement en utilisant les mêmes compositions vaccinales (i.e. les mêmes virus vaccinaux) et de préférence dans les mêmes conditions d'administrations (sites anatomiques et voies d'administration) que celles utilisées pour les 1$^{ère}$ et 2$^{ème}$ séries d'administrations.

Les phénomènes d'interférence peuvent s'expliquer par la dominance d'un ou plusieurs sérotypes par rapport à d'autres et sont donc indépendants de la technologie utilisée pour la fabrication du candidat vaccin (ex VDV ou ChimeriVax). La méthode selon la présente invention peut donc s'appliquer de façon générale à tout virus vaccinal de la dengue.

La présente invention a donc également pour objet l'utilisation de doses de virus vaccinal de la dengue pour la préparation d'un vaccin pour induire une protection contre les 4 sérotypes de la dengue comprenant :

(a) une première série d'administrations (i) d'une dose d'un virus vaccinal de la dengue d'un premier sérotype et d'une dose d'un virus vaccinal de la dengue d'un deuxième sérotype, et (ii) d'une dose d'un virus vaccinal de la dengue d'un troisième sérotype et d'une dose d'un virus vaccinal de la dengue d'un quatrième sérotype et
(b) une deuxième série d'administrations des doses (i) et (ii),

dans laquelle les doses (i) et (ii) sont administrées simultanément à des sites anatomiques distincts, et
dans laquelle la deuxième série est mise en oeuvre au moins 30 jours à au plus 12 mois après la première série.

Pour un descriptif des virus vaccinaux de la dengue utilisables dans le cadre de la présente invention on peut se reporter au descriptif qui en est donné en relation avec la méthode d'immunisation selon l'invention.

La présente invention a également pour objet un kit d'immunisation contre les quatre sérotypes du virus de la dengue. Le kit selon la présente invention comprend les doses telles que définies ci-dessus en relation avec la méthode d'immunisation proposée. Le kit selon l'invention comprend donc un boitier renfermant les différents récipients contenant les doses vaccinales et avantageusement une brochure explicative renfermant les informations utiles pour l'administration des vaccins.

Selon un mode de réalisation, le kit selon l'invention comprend un boitier contenant au moins les virus vaccinaux de la dengue des sérotypes 1, 2, 3 et 4

(a) sous la forme de compositions monovalentes contenues dans 4 récipients séparés, ou
(b) sous la forme de deux compositions bivalentes contenues dans 2 récipients séparés.

Selon un autre mode de réalisation, le kit selon l'invention comprend un boitier contenant au moins les virus vaccinaux de la dengue d'un premier et d'un deuxième sérotype,

(a) sous la forme de 2 compositions monovalentes contenues dans 2 récipients séparés, ou
(b) sous la forme d'une composition bivalente contenue dans 1 seul récipient.

Pour un descriptif des virus vaccinaux de la dengue utilisables dans le kit selon l'invention, on peut se référer à la description des virus vaccinaux donnée ci-dessus en relation avec la méthode d'immunisation selon l'invention.

Selon un mode de réalisation particulier, le kit selon la présente invention comprend donc au moins :

(a) un premier récipient contenant un vaccin bivalent comprenant un ChimeriVax™ DEN-1 et un ChimeriVax™ DEN-2, et

(b) un deuxième récipient contenant un vaccin bivalent comprenant un ChimeriVax™ DEN-3 et un ChimeriVax™ DEN-4.

Selon un autre mode de réalisation, le kit selon l'invention comprend au moins :

(a) un premier récipient contenant un vaccin bivalent comprenant un ChimeriVax™ DEN-1 et un ChimeriVax™ DEN-3, et

(b) un deuxième récipient contenant un vaccin bivalent comprenant un ChimeriVax™ DEN-2 et un ChimeriVax™ DEN-4.

Selon un autre mode de réalisation, le kit selon l'invention comprend au moins :

(a) un récipient contenant un vaccin bivalent comprenant un ChimeriVax™ DEN-1 et un ChimeriVax™ DEN-3, ou
(b) un récipient contenant un vaccin bivalent comprenant un ChimeriVax™ DEN-2 et un ChimeriVax™ DEN-4, ou
(c) un récipient contenant un vaccin bivalent comprenant un ChimeriVax™ DEN-1 et un ChimeriVax™ DEN-2, ou
(d) un récipient contenant un vaccin bivalent comprenant un ChimeriVax™ DEN-3 et un ChimeriVax™ DEN-4.

Les kits selon l'invention peuvent contenir un exemplaire unique ou plusieurs exemplaires des récipients tels que décrits ci-dessus.

Si les vaccins utilisés se présentent sous forme lyophilisée, le kit comprendra avantageusement au moins un récipient supplémentaire contenant le diluant permettant de reconstituer une dose vaccinale injectable. Tout diluant pharmaceutiquement acceptable peut être utilisé pour ce faire, classiquement, de l'eau ou une solution aqueuse tamponnée au phosphate.

L'invention est illustrée par les exemples suivants.

**<u>EXEMPLES</u>**

**<u>Exemple 1</u> : Immunisation par injection simultanée de deux compositions bivalentes en des sites anatomiques distincts chez le singe**

La virémie et l'immunogénicité ont été testées dans un modèle singe. La virémie, en particulier, a été identifiée comme l'un des facteurs associés avec la virulence et la sévérité de la maladie chez les hommes et constitue donc un paramètre important à prendre en considération. L'immunogénicité est quant à elle un paramètre clé dans le cadre de l'évaluation de la protection conférée.

1.1 <u>Matériels et méthodes :</u>

Les expériences chez les singes ont été menées selon les Directives européennes relatives à l'expérimentation animale. Les immunisations ont été effectuées chez des singes cynomolgus (*Macaca fascicularis*) originaires de Mauritanie. Les singes ont été mis en quarantaine pendant six semaines avant immunisation.

Les singes ont été immunisés par voie sous-cutanée dans les bras avec 0,5 ml de composition vaccinale. Après une légère anesthésie avec de la kétamine (Imalgene, Merial), du sang a été collecté par ponction au niveau des veines inguinale ou saphène. Aux jours 0 et 28 suivant chaque immunisation, 5 ml de sang ont été échantillonnés pour évaluer les réponses anticorps, alors qu'entre les jours 2 et 10, 1 ml de sang était échantillonné pour évaluer la virémie. Le sang était collecté sur la glace et conservé sur la glace jusqu'à séparation du sérum. Pour ce faire, le sang a été centrifugé pendant 20 minutes à 4°C et le sérum collecté stocké à -80°C jusqu'au moment des tests.

Mesure de la virémie

Les virémies post vaccinales ont été suivies par RT-PCT quantitative en temps réel (qRT-PCR). Deux jeux d'amorces et de sondes localisées dans le gène NS5 de des souches DEN1 et DEN2 ont été utilisés pour quantifier l'ARN de VDV-1 et VDV-2 respectivement. Un troisième jeu de 2 amorces et 1 sonde localisées dans le gène NS5 du virus YF a été utilisé pour quantifier l'ARN du CYD. Enfin, 4 jeux d'amorces et de sondes spécifiques des différents sérotypes CYD, localisées à la jonction des gènes E (DEN) / NS1 (YF) ont été utilisés pour identifier le sérotype dans les échantillons positifs pour l'ARN NS5 YF (voir aussi tableau I). 7 plasmides contenant, sous le contrôle du promoteur T7, la région ciblée par chaque PCR, ont été transcrits *in vitro* pour générer une série d'ARN synthétiques qui ont été inclus comme référence interne dans chaque essai de RT-PCT. Ces ARN synthétiques ont été dosés par spectrophotométrie, la quantité d'ARN obtenue a été convertie en nombre de copies d'ARN et exprimée en GEQ (équivalents génomiques).

0,140 ml de sérum de singe a été extrait à l'aide du kit d'extraction d'ARN « Nucleospin 96 virus™ » de Macherey Nagel, selon les instructions du fabriquant, puis l'ARN purifié a été élué avec 0,140 ml (0,090 ml, puis 0,05 ml) d'eau exempte de RNase. Pour éviter des cycles répétés de congélation/décongélation, une première quantification a été réalisée immédiatement après l'extraction sur 5 µl de ladite préparation d'ARN. Le volume restant a été congelé à 70°C.

Les mélanges réactionnels contenaient, en plus des composants du kit de quantification RT-PCR « Qiagen Qauntitect™probes » (Qiagen), 10 picomoles de chaque amorce, 4 picomoles de chaque sonde et 5 µl d'ARN, dans un volume total de 25 µl. Dans le cas des ARN à tester, 5 µl de la préparation purifiée ont été directement introduits dans le mélange réactionnel, sans étape de dilution préalable. Les ARN synthétiques ont été dilués au 1/10 dans de l'eau exempte de RNAse, et 7 dilutions contenant approximativement 10 à $10^6$ GEQ dans 5 µl ont été quantifiées en parallèle afin de générer la courbe étalon.

Les réactions de quantification ont été réalisées par l'appareil ABIPrism 700™ d'Applied Biosystem, en utilisant le programme suivant : 50°C/30 min, 95°C/15 min, puis 40 cycles de 95°C/15 sec-60°C/60 sec.

La limite de quantification de l'ARN viral dans ce test est de 2,9 à 3,3 $log_{10}$GEQ/ml (800 à 2000 GEQ/ml; 4 à 10 GEQ/réaction), selon les cibles PCR (déviation standard : +/-0,3 $log_{10}$)

La corrélation entre le titre infectieux et la quantification d'ARN viral a été établie parallèlement aux essais, par analyse de 0,140 ml d'échantillons de sérums de singe négatifs (DO) dans lesquels on a ajouté une quantité connue de particules infectieuses des virus ayant servi à l'immunisation (CYD ou VDV). Lesdits sérums contrôles ont été préparés à deux dilutions contenant environ 1 PFU et environ 100PFU dans 5 µl (2.3 and 4.3 $log_{10}$PFU/ml, respectivement).

Dans les tests utilisés dans les exemples la corrélation entre GEQ et PFU est la suivante : Ratio GEQ/PFU de 2.7 $log_{10}$ (soit : 1 PFU = 500 GEQ) pour les sera positifs en YF ou CYDs. Ratio GEQ/PFU de 2.5 $log_{10}$ (soit : 1 PFU = 320 GEQ) pour les sera positifs en VDV1 ou VDV2.

Les Limites de quantification étant < 3.3 $log_{10}$GEQ/ml (soit : <4 PFU/ml) pour les qRT-PCR YF et CYDs et < 2.9 $log_{10}$GEQ/ml (soit: < 2.5 PFU/ml) pour les qRT-PCR VDV1 et VDV2.

Les amorces et sondes utilisées sont données dans le tableau 1 ci-dessous dans lequel sont listés dans l'ordre pour chaque essai les amorces sens et anti-sens et la sonde.

Tableau 1

| | | sequence |
|---|---|---|
| YF | YF-NS5 sens | 5' GCACGGATGTAACAGACTGAAGA (23 bases) |
| | YF NS5 anti | 5' CCAGGCCGAACCTGTCAT (18 bases) |
| | YF-NS5 | 5' Fam- CGACTGTGTGGTCCGGCCCATC -Tamra (22 bases) |
| CYD1 spe | CYD1- sens | 5' CAT TGC AGT TGG CCT GGT AA (20 b) |
| | CYD1- antis | 5' CTT TGG CAA GAG AGA GCT CAA GT (23 b) |
| | CYD1- | 5' Fam-CCG ATC AAG GAT GCG CCA TCA-Tamra (21 b) |
| CYD2 spe | CYD2- sens | 5' GTG GGA GTC GTG ACG CTG TA (20 b) |
| | CYD2- anti | 5' GTT GAT GGC GCA TCC TTG ATC (21 b) |
| | CYD2 | 5' Fam-TGG GAG TTA TGG TGG GCG CCG-Tamra (21 b) |
| CYD3 spe | CYD3- sens | 5' AAA ACA CTT CCA TGT CAT TTT CAT G (25b) |
| | CYD3- anti | 5' GTT GAT GGC GCA TCC TTG ATC (21 b) |
| | CYD3- | 5'Fam-TGCGATAGGAATTATCACACTCTATCTGGGAGC-Tamra (33b) |
| CYD4 spe | CYD4- sens | 5' CTT AGT ATT GTG GAT TGG CAC GAA (24 b) |
| | CYD4- anti | 5' GCG CCA ACT GTG AAA CCT AGA (21 b) |
| | CYD4- | 5'-Fam-AGAAACACTTCAATGGCAATGACGTGCAT-Tamra (29 b) |
| VDV1 spe | VDV1-NS5 sens | 5' TCG CAA CAG CCT TAA CAG C (19 b) |
| | VDV1-NS5 anti | 5' ACT ATC TCC CTC CCA TCC TTC (21 b) |
| | VDV1-NS5 | 5' Fam-TTC ACA CCA CTT CCA C-MGB/NFQ (16 b) |
| VDV2 spec | VDV2-NS5 sens | 5' AAT GAC AGA CAC GAC TCC (18 b) |
| | VDV2-NS5 anti | 5' CCC AAA ACC TAC TAT CTT CAA C (22 b) |
| | VDV2-NS5 | 5' Fam-TGG AAG TCG GCA CGT GA-MGB/NFQ (17 b) |

Mesure des anticorps neutralisants (test de seroneutralisation) (SN50)) Classiquement, la mesure des anticorps Dengue est établie à l'aide du test PRNT50 (test de neutralisation par réduction à 50% du nombre de PFU). Ce test étant lourd et consommateur de matériel, nous avons développé le test SN50, basé sur la réduction à 50 % du nombre d'unités mesurées en test DICC50.

Dans une plaque de 96 puits, 0,120 ml de chaque sérum décomplémenté est ajouté à 0,480 ml de diluant (ISCOVE 4% SVF) par puits. Des dilutions en série d'un facteur 6 sont réalisées par transfert de 0,150 ml de sérum dans 0,450 ml de diluant. 450 $\mu$l de dilution virale à 2,7 $log_{10}$ DICC50/ml sont ajoutés dans chaque puits de façon à obtenir 25 DICC50/puits. La plaque est incubée à 37°C pendant 1 heure. 0.1 ml de chaque dilution est ensuite distribué dans 6 puits d'une plaque de 96 puits dans laquelle des cellules VERO ont été ensemencées 3 jours avant le début de l'expérience à une densité de 8000 cellules/puits, dans 0.1 ml de milieu ISCOVE 4% SVF,. Après 6 jours d'incubation à 37°C, en présence de 5% de $CO_2$, les cellules sont fixées à l'aide d'un mélange éthanol/acétone (70/30) à 4°C pendant 15 minutes, puis lavées à 3 reprises dans du PBS et incubées pendant 1 h à 37°C en présence de 0.05 ml d'une dilution au 1/2000 d'un anticorps monoclonal anti-flavivirus (mAb 4G2). Les plaques sont ensuite lavées à 2 reprises et incubées 1H à 37°C en présence de 0.05 ml d'une dilution au 1/1000 d'une IgG anti-souris conjuguée à la phosphatase alcaline. Les plages de lyse sont révélées par addition de 0.05 ml d'un substrat coloré : BCIP/NBT. Les titres en anticorps neutralisants sont calculés en utilisant la formule de Karber telle que définie ci-dessous :

$$log_{10}SN50 = d + f/N\ (X + N/2),$$

Dans laquelle:

- d : représente la dilution conduisant à 100% de neutralisation (soit 6 réplicats négatifs c'est-à-dire ne présentant pas de signe d'infection)
- f : représente le facteur de dilution en log10 (e.g. facteur de dilution de 1:4, f = 0,6)
- N: représente le nombre de réplicats / dilution (N=6)
- X: nombre total de puits ne présentant aucun signe d'infection, à l'exception de la dilution d.

La limite de détection virale est de 10 SN50 (i .e. 1.0 $log_{10}$SN50).

Les souches virales qui ont été utilisées pour la neutralisation sont les souches DEN1 16007, DEN2 16681, DEN3 16562 or DEN4 1036.

Pour les contrôles, les dilutions virales initiales ont été re-titrées.

La corrélation entre le titre neutralisant mesuré en test SN50 et le titre neutralisant mesuré classiquement en test PRNT50 est : $log_{10}$PRNT50 = $log_{10}$SN50 + 0,2.

Le titre moyen (GMT) est établi en calculant la moyenne géométrique des titres exprimés en valeur linéaire, les échantillons dont le titre est inférieur au seuil de détection se voient attribuer, par convention, une valeur égale à la moitié de ce seuil.

1.2 <u>Evaluation des immunisations simultanées</u>

2 groupes de 4 singes d'âge et poids équivalents ont été immunisés.

(voir tableau 2)

L'immunisation a été effectuée par voie sous-cutanée dans le bras avec une aiguille 23G1, à une dose $10^5$ $DICC_{50}$ pour chaque sérotype pour les vaccins CYD DEN 1 à 4.

Tableau 2 : Composition des groupes et protocole d'immunisation

| Singes | | |
|---|---|---|
| **Groupe** | **Immunisations** | |
| | **J0** | **J58** |
| **1** | **CYD 1 2** dans un bras<br>**CYD 3 4** dans l'autre bras | **CYD 1 2** dans un bras<br>**CYD 3 4** dans l'autre bras |
| **2** | **CYD 1234** | **CYD 1234** |

Les résultats d'immunogénicité obtenus après une immunisation (J28) et deux immunisations (J86) sont présentés dans le tableau 3.

Les résultats de virémie sont donnés dans le tableau 4.

Tableau 3 : titre neutralisant SN50 (unités 1/dil)

| Singes | | | | J0+28 | | | | J58+28 | | | |
|---|---|---|---|---|---|---|---|---|---|---|---|
| Groupe | ID | Immunisations J0 | Immunisations J58 | DEN-1 | DEN-2 | DEN-3 | DEN-4 | DEN-1 | DEN-2 | DEN-3 | DEN-4 |
| 1 | AP545 | **CYD 1 2** dans un bras **CYD 3 4** dans l'autre bras | **CYD 1 2** dans un bras **CYD 3 4** dans l'autre bras | 20 | 25 | - | 50 | 40 | 50 | - | 319 |
| | AO949 | | | 20 | - | - | 20 | 319 | 20 | 13 | 100 |
| | AP335 | | | 20 | - | - | 252 | 100 | 16 | 10 | 200 |
| | AP817 | | | 20 | 16 | 32 | 40 | 319 | 25 | 32 | 402 |
| | Moy géométrique | | | 20 | 10 | 8 | 56 | 142 | 25 | 12 | 225 |
| 2 | AP676 | CYD 1234 | CYD 1234 | 63 | - | - | 126 | 100 | - | - | 40 |
| | AQ005 | | | 25 | - | - | 63 | 50 | - | - | 63 |
| | AP961 | | | 50 | - | - | 158 | 80 | - | 80 | 400 |
| | AQ163 | | | 63 | - | - | 40 | 100 | - | 16 | 252 |
| | Moy géométrique | | | 47 | < 10 | <10 | 84 | 80 | < 10 | 13 | 126 |

- : titre < 10

Tableau 4 : analyse des virémies (unités :log10 GEQ/mL)

| | | Primoimmunisation | | | | | | | | Rappel | | | | | | |
|---|---|---|---|---|---|---|---|---|---|---|---|---|---|---|---|---|
| Groupe | Singe | J2 | J3 | J4 | J6 | J7 | J8 | J9 | J10 | J58 | J59 | J62 | J63 | J64 | J65 | J66 |
| **1** | AP545 | 3,17 | - | - | - | 3,12 | **3,67** | **4,55** | **4,59** | - | - | - | - | - | - | - |
| CYD1,2 + CYD3,4 | A0949 | **3,93** | **3,66** | 3,34 | **4,57** | - | - | - | - | - | - | - | - | - | - | - |
| 2pts injection | AP335 | **3,36** | 3,06 | **3,34** | **3,83** | **4,05** | **4,41** | **4,24** | **3,64** | - | - | - | - | - | - | - |
| | AP817 | *4,17* | **3,99** | **3,61** | - | - | - | - | 2,89 | - | - | - | - | - | - | - |
| **2** | AP676 | - | - | - | - | - | - | **3,65** | - | - | - | - | - | - | - | - |
| CYD 1,2,3,4 | AQ005 | **3,19** | - | 3,35 | - | - | - | - | **3,41** | - | - | - | - | - | - | - |
| 1 pt d'injection | AP961 | - | - | - | - | **3,86** | **3,42** | **3,29** | **3,56** | - | - | - | - | - | - | - |
| | AQ163 | **3,18** | **3,16** | - | **3,30** | **3,60** | 2,95 | 3,00 | - | - | - | - | - | - | - | - |

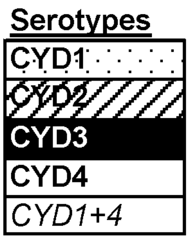


Brièvement, les résultats peuvent être résumés comme suit :

- Le schéma d'administration selon la présente invention permet d'augmenter qualitativement et quantitativement la réponse anticorps neutralisante homologue qui est obtenue avec la vaccination tétravalente.
- L'immunisation bivalente CYD-1,2 concomitante à une immunisation CYD-3,4 conduite à un site anatomique distinct induit après rappel des réponses homologues contre les quatre sérotypes chez tous les singes, sauf pour le serotype 3 chez un animal.
- De plus, les réponses contre les sérotypes 1 et 4 ont une tendance à être plus élevées dans le cas d'immunisations bivalentes simultanées que lors de l'immunisation tétravalente en un seul site.
- La virémie (tableau 4) est causée de manière prédominante par CYD-4, que ce soit après administration bivalente simultanée ou tétravalente. On peut donc en conclure que la séparation des sérotypes ne favorise pas l'émergence d'une virémie des sérotypes 1, 2 et 3.

Les exemples montrent donc que la méthode d'immunisation selon la présente invention améliore l'immunogénicité des virus vaccinaux de la dengue sans altérer la sécurité de ces derniers.

**Exemple 2 Immunisation par injection simultanée de deux compositions bivalentes CYD-1,4 et CYD-2,3 en des sites anatomiques distincts chez le singe**

La virémie et l'immunogénicité ont été testées dans le modèle singe comme dans l'exemple 1. Dans le présent exemple, les compositions bivalentes testées contiennent respectivement les virus vaccinaux les plus immunogènes (CYD-1,4) et les virus vaccinaux les moins immunogènes (CYD-2,3).

2.1 Matériels et méthodes : identiques à l'exemple 1

2.2 Evaluation des immunisations simultanées

2 groupes de 4 singes d'âge et poids équivalents ont été immunisés. (voir tableau 5)
L'immunisation a été effectuée comme décrit dans l'exemple 1

Tableau 5 : Composition des groupes et protocole d'immunisation

| Singes | | |
|---|---|---|
| Groupe | Immunisations | |
| | J0 | J58 |
| 1 | **CYD 1 4** dans un bras<br>**CYD 2 3** dans l'autre bras | **CYD 1 4** dans un bras<br>**CYD 2 3** dans l'autre bras |
| 2 | **CYD 1234** | **CYD 1234** |

Les résultats d'immunogénicité obtenus après une immunisation (J28) et deux immunisations (J86) sont présentés dans le tableau 6.
Les résultats de virémie sont similaires à ceux obtenus dans l'exemple 1, montrant une virémie induite par le sérotype 4 et pas de différences significatives entre les deux groupes

Tableau 6 : titre neutralisant SN50 (unités 1/dil)

| Singges | | | | J0+28 | | | | J58+28 | | | |
|---|---|---|---|---|---|---|---|---|---|---|---|
| **Groupe** | **ID** | **Immunisations** | | **DEN-1** | **DEN-2** | **DEN-3** | **DEN-4** | **DEN-1** | **DEN-2** | **DEN-3** | **DEN-4** |
| | | **J0** | **J58** | | | | | | | | |
| **1** | AR465 | **CYD 1234** | **CYD 1234** | 63 | 10 | < | 100 | 200 | 25 | 10 | 126 |
| | AR558 | | | 13 | < | < | 126 | 401 | < | 20 | 160 |
| | AR559 | | | < | < | < | 100 | 13 | < | < | 50 |
| | AR639 | | | 25 | < | < | 318 | 201 | < | < | 201 |
| | Moyenne géométrique | | | **20** | < | < | **142** | **119** | < | < | **119** |
| **2** | AR083 | **CYD 1 4** dans un bras | **CYD 1 4** dans un bras | 63 | < | < | 201 | 100 | 16 | 10 | 126 |
| | AR506 | | | 63 | 25 | 13 | 638 | 126 | 100 | 32 | 201 |
| | AR610 | **CYD 2 3** dans l'autre bras | **CYD 2 3** dans l'autre bras | 63 | 20 | < | 100 | 159 | 50 | 40 | 253 |
| | AR644 | | | 40 | < | < | 40 | 505 | 80 | 40 | 100 |
| | Moyenne géométrique | | | **56** | < | < | **150** | **178** | **50** | **27** | **159** |

< : titre < 10

Les résultats confortent ceux obtenus dans l'exemple 1 et peuvent être résumés comme suit :

- Le schéma d'administration permet d'augmenter qualitativement et quantitativement la réponse anticorps neutralisante homologue qui est obtenue avec la vaccination tétravalente.
- L'immunisation bivalente CYD-1,4 concomitante à une immunisation CYD-2,3 conduite à un site anatomique distinct induit après rappel des réponses homologues contre les quatre sérotypes chez tous les singes, ce qui n'est pas le cas dans le groupe tétravalent classique, comme vu dans l'exemple 1.
- Comparés à ceux du groupe de singes ayant reçu deux bivalents CYD-1,2 et CYD-3,4 dans l'exemple 1, les niveaux anticorps observés après immunisation bivalente CYD-1,4 concomitante à une immunisation CYD-2,3 sont supérieurs pour les sérotypes 1, 2 et 3, et inférieurs pour le sérotype 4, ce qui montre une réponse mieux équilibrée entre les 4 sérotypes, avec une dominance moindre du 4.
- La séparation des sérotypes dominants des autres dans un tel schéma d'immunisation a permis une réponse équilibrée contre les 4 sérotypes

Les 2 exemples ci-dessus montrent donc que la méthode d'immunisation selon la présente invention améliore l'immunogénicité des virus vaccinaux de la dengue sans altérer la sécurité de ces derniers telle qu'évaluée par la mesure de la virémie.

LISTAGE SEQUENCES

<110> Sanofi Pasteur
Sanofi Pasteur

<120> Méthode d'immunisation contre les 4 sérotypes de la dengue
<130> PM0607
<160> 2
<170> PatentIn version 3.3
<210> 1
<211> 10735
<212< DNA
<213> Dengue virus
<400> 1

agttgttagt ctacgtggac cgacaagaac agtttcgaat cggaagcttg cttaacgtag 60
ttctaacagt tttttattag agagcagatc tctgatgatc aaccaacgaa aaaagacggg 120
tcgaccgtct ttcaatatgc tgaaacgcgc gagaaaccgc gtgtcaactg tttcacagtt 180
ggcgaagaga ttctcaaaag gattgctctc aggccaagga cccatgaaat tggtgatggc 240
tttcatagca ttcttaagat ttctagccat acccccaaca gcaggaattt tggctagatg 300
gggctcattc aagaagaatg gagcgattaa agtgttacgg ggtttcaaga gagaaatctc 360
aaacatgcta aacataatga acaggaggaa aagatccgtg accatgctcc ttatgctgct 420
gcccacagcc ctggcgttcc atctgacgac acgaggggga gagccgcata tgatagttag 480
caagcaggaa agaggaaagt cacttttgtt caagacctct gcaggtgtca acatgtgcac 540
cctcattgcg atggatttgg gagagttgtg tgaggacacg atgacctaca aatgcccccg 600
gatcactgag gcggaaccag atgacgttga ctgttggtgc aatgccacgg acacatgggt 660
gacctatgga acgtgctctc aaactggcga acaccgacga gacaaacgtt ccgtcgcatt 720
ggccccacac gtggggcttg gcctagaaac aagagccgaa acgtggatgt cctctgaagg 780
tgcttggaaa cagatacaaa aagtagagac ttgggctctg agacatccag gattcacggt 840
gatagccctt tttctagcac atgccatagg aacatccatc acccagaaag ggatcatttt 900
cattttgctg atgctggtaa caccatctat ggccatgcga tgcgtgggaa taggcaacag 960
agacttcgtg gaaggactgt caggagcaac atgggtggat gtggtactgg agcatggaag 1020
ttgcgtcacc accatggcaa aaaacaaacc aacactggac attgaactct tgaagacgga 1080
ggtcacaaac cctgcagttc tgcgtaaatt gtgcattgaa gctaaaatat caaacaccac 1140

caccgattcg agatgtccaa cacaaggaga agccacactg gtggaagaac aagacgcgaa 1200
ctttgtgtgc cgacgaacgt tcgtggacag aggctggggc aatggctgtg ggctattcgg 1260
aaaaggtagt ctaataacgt gtgccaagtt taagtgtgtg acaaaactag aaggaaagat 1320
agctcaatat gaaaacctaa aatattcagt gatagtcacc gtccacactg gagatcagca 1380
ccaggtggga aatgagacta cagaacatgg aacaactgca accataacac ctcaagctcc 1440
tacgtcggaa atacagctga ccgactacgg aacccttaca ttagattgtt cacctaggac 1500
agggctagat tttaacgaga tggtgttgct gacaatgaaa aagaaatcat ggcttgtcca 1560
caaacagtgg tttctagact taccactgcc ttggacctct ggggctttaa catcccaaga 1620
gacttggaac agacaagatt tactggtcac atttaagaca gctcatgcaa agaagcagga 1680
agtagtcgta ctaggatcac aagaaggagc aatgcacact gcgctgactg gagcgacaga 1740
aatccaaacg tcaggaacga caacaatttt cgcaggacac ctaaaatgca gactaaaaat 1800
ggacaaacta actttaaaag ggatgtcata tgtgatgtgc acaggctcat tcaagttaga 1860
gaaagaagtg gctgagaccc agcatggaac tgttctggtg caggttaaat atgaaggaac 1920
agacgcacca tgcaagattc ccttttcgac ccaagatgag aaaggagcaa cccagaatgg 1980
gagattaata acagccaacc ccatagtcac tgacaaagaa aaaccagtca atattgaggc 2040
agaaccaccc tttggtgaga gctacatcgt ggtaggagca ggtgaaaaag ctttgaaact 2100
aagctggttc aagaaaggaa gcagcatagg gaaaatgttt gaagcaactg cccgaggagc 2160
acgaaggatg gccattctgg gagacaccgc atgggacttc ggttctatag gaggagtgtt 2220
cacgtctatg ggaaaactgg tacaccaggt ttttggaact gcatatggag ttttgtttag 2280
cggagtttct tggaccatga aaataggaat agggattctg ctgacatggc taggattaaa 2340
ttcaaggaac acgtcccttt cggtgatgtg catcgcagtt ggcatggtca cactgtacct 2400
aggagtcatg gttcaggcag attcgggatg tgtaatcaac tggaaaggca gagaacttaa 2460
atgtggaagc ggcatttttg tcactaatga agttcacact tggacagagc aatacaaatt 2520
ccaggctgac tcccccaaga gactatcagc agccattggg aaggcatggg aggagggtgt 2580
gtgtggaatc cgatcagcca ctcgtctcga gaacatcatg tggaaacaaa tatcaaatga 2640
attgaaccac atcctacttg aaaatgacat gaaatttaca gtggtcgtgg gagacgttag 2700
tggaatcttg gcccaaggaa aaaaaatgat taggccacaa cccatggaac acaaatactc 2760
gtggaaaagc tggggaaaag ctaaaatcat aggagcggat gtacagaaca ccaccttcat 2820
catcgacggc ccaaacaccc cagaatgccc tgacaatcaa agagcatgga atatttggga 2880
agtagaggac tatggatttg ggattttcac gacaaacata tggttgaaat tgcgtgactc 2940
ctacacccaa gtatgtgacc accggctgat gtcagctgcc attaaggaca gcaaggcagt 3000
ccatgctgac atggggtact ggatagaaag tgaaaagaac gagacatgga agttggcgag 3060
agcctccttt atagaagtta agacatgcat ctggccaaaa tcccacactc tatggagcaa 3120

tggagttctg gaaagtgaaa tgataattcc aaagatatat ggaggaccaa tatctcagca 3180
caactacaga ccaggatatt tcacacaaac agcagggccg tggcacctag gcaagttgga 3240
actagatttc gatttttgtg aaggtaccac agttgttgtg gatgaacatt gtggaaatcg 3300
aggaccatct ctcagaacca caacagtcac aggaaagata atccatgaat ggtgctgcag 3360
atcttgtacg ctacccccc tacgtttcaa aggggaagac gggtgttggt acggcatgga 3420
aatcagacca gtgaaggaca aggaagagaa cctggtcaag tcaatggtct ctgcagggtc 3480
aggagaagtg gacagctttt cactaggact gctatgcata tcaataatga ttgaagaagt 3540
gatgagatcc agatggagca aaaaaatgct gatgactgga acactggctg tgttcctcct 3600
tcttataatg ggacaattga catggagtga tctgatcagg ttatgtatta tggttggagc 3660
caacgcttca gacaagatgg ggatgggaac aacgtaccta gctttaatgg ccactttcaa 3720
aatgagacca atgttcgccg tcgggctatt atttcgcaga ctaacatcta gagaagttct 3780
tcttcttaca attggcttga gcctggtggc atccgtggag ctaccaagtt ccctagagga 3840
gctgggggat ggacttgcaa taggcatcat gatgttgaaa ttattgactg attttcagtc 3900
acaccagcta tgggctactc tgctatcctt gacatttatt aaaacaactt tttcattgca 3960
ctatgcatgg aagacaatgg ctatggtact gtcaattgta tctctcttcc ctttatgcct 4020
gtccacgacc tctcaaaaaa caacatggct tccggtgctg ttgggatctc ttggatgcaa 4080
accactaccc atgtttctta taacagaaaa caaaatctgg ggaaggaaga gttggcccct 4140
caatgaagga attatggctg ttggaatagt tagtattcta ctaagttcac ttttaaaaaa 4200
tgatgtgccg ctagccggcc cattaatagc tggaggcatg ctaatagcat gttatgtcat 4260
atccggaagc tcagctgatt tatcactgga gaaagcggct gaggtctcct gggaggaaga 4320
agcagaacac tcaggcgcct cacacaacat actagtagag gttcaagatg atggaaccat 4380
gaagataaaa gatgaagaga gagatgacac gctcaccatt ctccttaaag caactctgct 4440
ggcagtctca ggggtgtacc caatgtcaat accagcgacc ctttttgtgt ggtatttttg 4500
gcagaaaaag aaacagagat caggagtgct atgggacaca cccagccccc cagaagtgga 4560
aagagcagtt cttgatgatg gcatctatag aattttgcaa agaggactgt tgggcaggtc 4620
ccaagtagga gtaggagttt tccaagaagg cgtgttccac acaatgtggc acgtcactag 4680
gggagctgtc ctcatgtatc aaggaaaaag gctggaacca agctgggcca gtgtcaaaaa 4740
agacttgatc tcatatggag gaggttggag gtttcaagga tcctggaaca cgggagaaga 4800
agtacaggtg attgctgttg aaccgggaaa aaaccccaaa aatgtacaaa caacgccggg 4860
taccttcaag acccctgaag gcgaagttgg agccatagcc ttagacttta aacctggcac 4920
atctggatct cccatcgtaa acagagaggg aaaaatagta ggtctttatg gaaatggagt 4980
ggtgacaaca agcggaactt acgttagtgc catagctcaa gctaaggcat cacaagaagg 5040
gcctctacca gagattgagg acaaggtgtt taggaaaaga aacttaacaa taatggacct 5100

acatccagga tcgggaaaaa caagaagata ccttccagcc atagtccgtg aggccataaa 5160
aaggaagctg cgcacgctaa tcctagctcc cacaagagtt gtcgcttctg aaatggcaga 5220
ggcactcaag ggagtgccaa taaggtatca gacaacagca gtgaagagtg aacacacagg 5280
aaaggagata gttgacctta tgtgccacgc cactttcacc atgcgcctcc tgtctcccgt 5340
gagagttccc aattataaca tgattatcat ggatgaagca cacttcaccg atccagccag 5400
catagcagcc agagggtaca tctcaacccg agtgggtatg ggtgaagcag ctgcgatctt 5460
tatgacagcc actcccccag gatcggtgga ggcctttcca cagagcaatg caattatcca 5520
agatgaggaa agagacattc ctgagagatc atggaactca ggctatgact ggatcactga 5580
ttttccaggt aaaacagtct ggtttgttcc aagcatcaaa tcaggaaatg acattgccaa 5640
ctgtttaaga aaaaacggga aacgggtgat ccaattgagc agaaaaacct ttgacactga 5700
gtaccagaaa acaaaaaaca acgactggga ctatgtcgtc acaacagaca tttccgaaat 5760
gggagcaaat ttccgggccg acagggtaat agacccaagg cggtgtctga aaccggtaat 5820
actaaaagat ggtccagagc gcgtcattct agccggaccg atgccagtga ctgtggccag 5880
tgccgcccag aggagaggaa gaattggaag gaaccaaaac aaggaaggtg atcagtatat 5940
ttacatggga cagcctttaa aaaatgatga ggaccacgct cattggacag aagcaaagat 6000
gctccttgac aatataaaca caccagaagg gattatccca gccctctttg agccggagag 6060
agaaaagagt gcagctatag acggggaata cagactgcgg ggtgaagcaa ggaaaacgtt 6120
cgtggagctc atgagaagag gggatctacc agtctggcta tcctacaaag ttgcctcaga 6180
aggcttccag tactccgaca gaaggtggtg cttcgatggg gaaaggaaca accaggtgtt 6240
ggaggagaac atggacgtgg agatctggac aaaagaagga gaaagaaaga aactacgacc 6300
tcgctggttg gacgccagaa catactctga cccactggct ctgcgcgagt ttaaagagtt 6360
tgcagcagga agaagaagcg tctcaggtga cctaatatta gaaataggga aacttccaca 6420
acatttgacg caaagggccc agaatgcttt ggacaacttg gtcatgttgc acaattccga 6480
acaaggagga aaagcctata gacatgctat ggaagaactg ccagacacaa tagaaacgtt 6540
gatgctccta gccttgatag ctgtgttgac tggtggagtg acgctgttct tcctatcagg 6600
aagaggtcta ggaaaaacat ctatcggctt actctgcgtg atggcctcaa gcgcactgtt 6660
atggatggcc agtgtggagc cccattggat agcggcctcc atcatactgg agttctttct 6720
gatggtactg cttattccag agccagacag acagcgcact ccacaggaca accagctagc 6780
atatgtggtg ataggtctgt tattcgtgat attgacagtg gcagccaatg agatgggatt 6840
attggaaacc acaaagaaag acctggggat tggccatgta gctgctgaaa accaccacca 6900
tgctacaatg ctggacgtag acctacatcc agcttcagcc tggaccctct atgcagtggc 6960
cacaacaatc atcactccta tgatgagaca cacaattgaa aacacaacgg caaatatttc 7020
cctgacagcc atcgcaaacc aagcagctat attgatggga cttgacaagg gatggccaat 7080

atcgaagatg gacataggag ttccacttct cgccttgggg tgctattccc aagtgaatcc 7140
gctgacactg atagcggcag tattgatgct agtagctcat tacgccataa ttggacctgg 7200
actgcaagca aaagctacta gagaagctca aaaaagaaca gcggctggaa taatgaaaaa 7260
tccaactgtc gacgggattg ttgcaataga cttagatccc gtggtttacg atgcaaaatt 7320
tgaaaaacag ctaggccaaa taatgttgtt gatactttgc acatcacaga ttcttttgat 7380
gcggactaca tgggccttgt gtgaatccat cacattggct actggacctc tgaccactct 7440
ttgggaggga tctccaggaa aattctggaa caccacaata gcggtatcca tggcaaacat 7500
tttcaggggg agttatctag caggagcagg tctggccttc tcattaatga aatctctagg 7560
aggaggtagg agaggcacgg gagcccaagg ggaaacactg ggagaaaaat ggaaaagaca 7620
actaaaccaa ctgagcaagt cagaattcaa tacttacaag aggagtggga ttatggaggt 7680
ggatagatcc gaagccaaag agggactgaa aagaggagaa acaaccaaac acgcagtatc 7740
gagaggaacg gccaaactga ggtggttcgt ggagaggaac cttgtgaaac cagaagggaa 7800
agtcatagac ctcggttgtg gaagaggtgg ctggtcatat tattgcgctg ggctgaagaa 7860
agtcacagaa gtgaaaggat acacaaaagg aggacctgga catgaggaac caatcccaat 7920
ggcgacctat ggatggaacc tagtaaggct gcactccgga aaagatgtat tttttatacc 7980
acctgagaaa tgtgacaccc ttttgtgtga tattggtgag tcctctccga acccaactat 8040
agaggaagga agaacgttac gtgttctgaa aatggtggaa ccatggctca gaggaaacca 8100
attttgcata aaaattctaa atccctatat gccgagcgtg gtagaaactc tggaacaaat 8160
gcaaagaaaa catggaggaa tgctagtgcg aaacccactc tcaagaaatt ccacccatga 8220
aatgtactgg gtttcatgtg gaacaggaaa cattgtgtca gcagtaaaca tgacatctag 8280
aatgttgcta aatcggttca caatggctca caggaagcca acatatgaaa gagacgtgga 8340
cttaggcgct ggaacaagac atgtggcagt agaaccagag gtagccaacc tagatatcat 8400
tggccagagg atagagaata taaaaaatga acataagtca acatggcatt atgatgagga 8460
caatccatac aaaacatggg cctatcatgg atcatatgag gttaagccat caggatcggc 8520
ctcatccatg gtcaatggcg tggtgagatt gctcaccaaa ccatgggatg ttatccccat 8580
ggtcacacaa atagccatga ctgataccac accctttgga caacagaggg tgtttaaaga 8640
gaaagttgac acgcgcacac caaaagcaaa acgtggcaca gcacaaatta tggaagtgac 8700
agccaggtgg ttatggggtt tcctttctag aaacaaaaaa cccagaattt gcacaagaga 8760
ggagtttaca agaaaagtta ggtcaaacgc agctattgga gcagtgttcg ttgatgaaaa 8820
tcaatggaac tcggcaaaag aagcagtgga agacgaacgg ttctgggaac ttgtccacag 8880
agagagggag cttcataaac aggggaaatg tgccacgtgt gtctacaata tgatggggaa 8940
gagagagaaa aaattaggag agttcggaaa ggcaaaagga agtcgtgcaa tatggtacat 9000
gtggttggga gcacgcttcc tagagtttga agcccttggt ttcatgaatg aagatcactg 9060

gttcagtaga gagaattcac tcagtggagt ggaaggagaa ggactccaca aacttggata 9120
catactcaga gacatatcaa ggattccagg ggggaacatg tatgcagatg acacagccgg 9180
atgggacaca agaataacag aggatgatct ccagaatgag gctaaaatca ctgacatcat 9240
ggagcccgaa catgccctgc tggctacgtc aatctttaag ctgacctacc aaaataaggt 9300
ggtaagggtg cagagaccag caaaaaatgg aaccgtgatg gatgttatat ccagacgtga 9360
ccagagaggc agtggacagg ttggaactta tggcttaaac actttcacca acatggaggc 9420
ccaactgata agacaaatgg agtctgaggg aatcttttta cccagcgaat tggaaacccc 9480
aaatctagcc ggaagagttc tcgactggtt ggaaaaatat ggtgtcgaaa ggctgaaaag 9540
aatggcaatc agcggagatg actgtgtggt gaaaccaatt gatgacaggt tcgcaacagc 9600
cttaacagct ttgaatgaca tgggaaaagt aagaaaagac ataccacaat gggaaccttc 9660
aaaaggatgg aatgattggc aacaagtgcc tttctgttca caccacttcc accagctaat 9720
tatgaaggat gggaggaga tagtggtgcc atgccgcaac caagatgaac ttgtggggag 9780
ggccagagta tcacaaggcg ccggatggag cctgagagaa accgcatgcc taggcaagtc 9840
atatgcacaa atgtggcagc tgatgtattt ccacaggaga gacctgagac tggcggctaa 9900
cgctatttgt tcagccgttc cagttgattg ggtcccaacc agccgcacca cctggtcgat 9960
ccatgcccat caccaatgga tgacaacaga agacatgtta tcagtatgga atagggtctg 10020
gatagaggaa aacccatgga tggaggataa gactcatgtg tccagttggg aagaagttcc 10080
atacctagga aagagggaag atcagtggtg tggatccctg ataggcttaa cagcaagggc 10140
cacctgggcc actaatatac aagtggccat aaaccaagtg agaaggctca ttgggaatga 10200
gaattatcta gattacatga catcaatgaa gagattcaag aatgagagtg atcccgaagg 10260
ggcactctgg taagtcaaca cattcacaaa ataaaggaaa ataaaaaatc aaatgaggca 10320
agaagtcagg ccagattaag ccatagtacg gtaagagcta tgctgcctgt gagccccgtc 10380
caaggacgta aaatgaagtc aggccgaaag ccacggtttg agcaagccgt gctgcctgtg 10440
gctccatcgt ggggatgtaa aaacccggga ggctgcaacc catggaagct gtacgcatgg 10500
ggtagcagac tagtggttag aggagacccc tcccaagaca caacgcagca gcggggccca 10560
acaccagggg aagctgtacc ctggtggtaa ggactagagg ttagaggaga ccccccgcgt 10620
aacaataaac agcatattga cgctgggaga gaccagagat cctgctgtct ctacagcatc 10680
attccaggca cagaacgcca gaaaatggaa tggtgctgtt gaatcaacag gttct 10735

<212> Type : DNA
<211> Length : 10735
SequenceName : vdv1
SequenceDescription :

Sequence

--------

<213> OrganismName : Dengue virus

<400> PreSequenceString :

agttgttagt ctacgtggac cgacaaagac agattctttg agggagctaa gctcaatgta 60
gttctaacag tttttaatt agagagcaga tctctgatga ataaccaacg gaaaaaggcg 120
aaaaacacgc ctttcaatat gctgaaacgc gagagaaacc gcgtgtcgac tgtgcaacag 180
ctgacaaaga gattctcact tggaatgctg cagggacgag gaccattaaa actgttcatg 240
gccctggtgg cgttccttcg tttcctaaca atcccaccaa cagcagggat attgaagaga 300
tggggaacaa ttaaaaaatc aaaagctatt aatgttttga gagggttcag gaaagagatt 360
ggaaggatgc tgaacatctt gaataggaga cgcagatctg caggcatgat cattatgctg 420
attccaacag tgatggcgtt ccatttaacc acacgtaacg gagaaccaca catgatcgtc 480
agcagacaag agaaagggaa aagtcttctg tttaaaacag aggttggcgt gaacatgtgt 540
accctcatgg ccatggacct tggtgaattg tgtgaagaca caatcacgta caagtgtccc 600
cttctcaggc agaatgagcc agaagacata gactgttggt gcaactctac gtccacgtgg 660
gtaacttatg ggacgtgtac caccatggga gaacatagaa gagaaaaaag atcagtggca 720
ctcgttccac atgtgcgaat gggactggag acacgaactg aaacatggat gtcatcagaa 780
ggggcctgga aacatgtcca gagaattgaa acttggatct tgagacatcc aggcttcacc 840
atgatggcag caatcctggc atacaccata ggaacgacac atttccaaag agccctgatt 900
ttcatcttac tgacagctgt cactccttca atgacaatgc gttgcatagg aatgtcaaat 960
agagactttg tggaaggggt ttcaggagga agctgggttg acatagtctt agaacatgga 1020
agctgtgtga cgacgatggc aaaaaacaaa ccaacattgg attttgaact gataaaaaca 1080
gaagccaaac agcctgccac cctaaggaag tactgtatag aggcaaagct aaccaacaca 1140
acaacagaat ctcgctgccc aacacaaggg gaacccagcc taaatgaaga gcaggacaaa 1200
aggttcgtct gcaaacactc catggtagac agaggatggg gaaatggatg tggactattt 1260
ggaaagggag gcattgtgac ctgtgctatg ttcagatgca aaaagaacat ggaaggaaaa 1320
gttgtgcaac cagaaaactt ggaatacacc attgtgataa cacctcactc aggggaagag 1380
catgcagtcg gaaatgacac aggaaaacat ggcaaggaaa tcaaaataac accacagagt 1440
tccatcacag aagcagaatt gacaggttat ggcactgtca caatggagtg ctctccaaga 1500
acgggcctcg acttcaatga gatggtgttg ctgcagatgg aaaataaagc ttggctggtg 1560
cacaggcaat ggttcctaga cctgccgtta ccatggttgc ccggagcgga cacacaagag 1620
tcaaattgga tacagaagga gacattggtc actttcaaaa atccccatgc gaagaaacag 1680

gatgttgttg ttttaggatc ccaagaaggg gccatgcaca cagcacttac aggggccaca 1740
gaaatccaaa tgtcatcagg aaacttactc ttcacaggac atctcaagtg caggctgaga 1800
atggacaagc tacagctcaa aggaatgtca tactctatgt gcacaggaaa gtttaaagtt 1860
gtgaaggaaa tagcagaaac acaacatgga acaatagtta tcagagtgca atatgaaggg 1920
gacggctctc catgcaagat cccttttgag ataatggatt tggaaaaaag acatgtctta 1980
ggtcgcctga ttacagtcaa cccaattgtg acagaaaaag atagcccagt caacatagaa 2040
gcagaacctc catttggaga cagctacatc atcataggag tagagccggg acaactgaag 2100
ctcaactggt ttaagaaagg aagttctatc ggccaaatgt ttgagacaac aatgaggggg 2160
gcgaagagaa tggccatttt aggtgacaca gcctgggatt ttggatcctt gggaggagtg 2220
tttacatcta taggaaaggc tctccaccaa gtctttggag caatctatgg agctgccttc 2280
agtggggttt catggactat gaaaatcctc ataggagtca ttatcacatg gataggaatg 2340
aattcacgca gcacctcact gtctgtgaca ctagtattgg tgggaattgt gacactgtat 2400
ttgggagtca tggtgcaggc cgatagtggt tgcgttgtga gctggaaaaa caaagaactg 2460
aaatgtggca gtgggatttt catcacagac aacgtgcaca catggacaga acaatacaaa 2520
ttccaaccag aatccccttc aaaactagct tcagctatcc agaaagccca tgaagaggac 2580
atttgtggaa tccgctcagt aacaagactg gagaatctga tgtggaaaca aataacacca 2640
gaattgaatc acattctatc agaaaatgag gtgaagttaa ctattatgac aggagacatc 2700
aaaggaatca tgcaggcagg aaaacgatct ctgcggcctc agcccactga gctgaagtat 2760
tcatggaaaa catggggcaa agcaaaaatg ctctctacag agtctcataa ccagaccttt 2820
ctcattgatg gccccgaaac agcagaatgc cccaacacaa atagagcttg gaattcgttg 2880
gaagttgaag actatggctt tggagtattc accaccaata tatggctaaa attgaaagaa 2940
aaacaggatg tattctgcga ctcaaaactc atgtcagcgg ccataaaaga caacagagcc 3000
gtccatgccg atatgggtta ttggatagaa agtgcactca atgacacatg gaagatagag 3060
aaagcctctt tcattgaagt taaaaactgc cactggccaa aatcacacac cctctggagc 3120
aatggagtgc tagaaagtga gatgataatt ccaaagaatc tcgctggacc agtgtctcaa 3180
cacaactata gaccaggcta ccatacacaa ataacaggac catggcatct aggtaagctt 3240
gagatggact ttgatttctg tgatggaaca acagtggtag tgactgagga ctgcggaaat 3300
agaggaccct ctttgagaac aaccactgcc tctggaaaac tcataacaga atggtgctgc 3360
cgatcttgca cattaccacc gctaagatac agaggtgagg atgggtgctg gtacgggatg 3420
gaaatcagac cattgaagga gaaagaagag aatttggtca actccttggt cacagctgga 3480
catgggcagg tcgacaactt ttcactagga gtcttgggaa tggcattgtt cctggaggaa 3540
atgcttagga cccgagtagg aacgaaacat gcaatactac tagttgcagt ttcttttgtg 3600
acattgatca cagggaacat gtcctttaga gacctgggaa gagtgatggt tatggtaggc 3660

gccactatga cggatgacat aggtatgggc gtgacttatc ttgccctact agcagccttc 3720
aaagtcagac caacttttgc agctggacta ctcttgagaa agctgacctc caaggaattg 3780
atgatgacta ctataggaat tgtactcctc tcccagagca ccataccaga gaccattctt 3840
gagttgactg atgcgttagc cttaggcatg atggtcctca aaatggtgag aaatatggaa 3900
aagtatcaat tggcagtgac tatcatggct atcttgtgcg tcccaaacgc agtgatatta 3960
caaaacgcat ggaaagtgag ttgcacaata ttggcagtgg tgtccgtttc cccactgttc 4020
ttaacatcct cacagcaaaa aacagattgg ataccattag cattgacgat caaaggtctc 4080
aatccaacag ctatttttct aacaaccctc tcaagaacca gcaagaaaag gagctggcca 4140
ttaaatgagg ctatcatggc agtcgggatg gtgagcattt tagccagttc tctcctaaaa 4200
aatgatattc ccatgacagg accattagtg gctggagggc tcctcactgt gtgctacgtg 4260
ctcactggac gatcggccga tttggaactg gagagagcag ccgatgtcaa atgggaagac 4320
caggcagaga tatcaggaag cagtccaatc ctgtcaataa caatatcaga agatggtagc 4380
atgtcgataa aaaatgaaga ggaagaacaa acactgacca tactcattag aacaggattg 4440
ctggtgatct caggactttt tcctgtatca ataccaatca cggcagcagc atggtacctg 4500
tgggaagtga agaaacaacg ggccggagta ttgtgggatg ttccttcacc cccacccatg 4560
ggaaaggctg aactggaaga tggagcctat agaattaagc aaaaagggat tcttggatat 4620
tcccagatcg gagccggagt ttacaaagaa ggaacattcc atacaatgtg gcatgtcaca 4680
cgtggcgctg ttctaatgca taaaggaaag aggattgaac caacatgggc ggacgtcaag 4740
aaagacctaa tatcatatgg aggaggctgg aagttagaag gagaatggaa ggaaggagaa 4800
gaagtccagg tattggcact ggagcctgga aaaaatccaa gagccgtcca aacgaaacct 4860
ggtcttttca aaaccaacgc cggaacaata ggtgctgtat ctctggactt ttctcctgga 4920
acgtcaggat ctccaattat cgacaaaaaa ggaaaagttg tgggtcttta tggtaatggt 4980
gttgttacaa ggagtggagc atatgtgagt gctatagccc agactgaaaa aagcattgaa 5040
gacaacccag agatcgaaga tcacattttc cgaaagagaa gactgaccat catggacctc 5100
cacccaggag cgggaaagac gaagagatac cttccggcca tagtcagaga agctataaaa 5160
cggggtttga gaacattaat cttggccccc actagagttg tggcagctga aatggaggaa 5220
gcccttagag gacttccaat aagataccag accccagcca tcagagctga gcacaccggg 5280
cgggagattg tggacctaat gtgtcatgcc acatttacca tgaggctgct atcaccagtt 5340
agagtgccaa actacaacct gattatcatg gacgaagccc atttcacaga cccagcaagt 5400
atagcagcta gaggatacat ctcaactcga gtggagatgg gtgaggcagc tgggattttt 5460
atgacagcca ctcccccggg aagcagagac ccatttcctc agagcaatgc accaatcata 5520
gatgaagaaa gagaaatccc tgaacgctcg tggaattccg gacatgaatg ggtcacggat 5580
tttaaaggga agactgtttg gttcgttcca agtataaaag caggaaatga tatagcagct 5640

tgcctgagga aaaatggaaa gaaagtgata caactcagta ggaagacctt tgattctgag 5700
tatgtcaaga ctagaaccaa tgattgggac ttcgtggtta caactgacat ttcagaaatg 5760
ggtgccaatt tcaaggctga gagggttata gaccccagac gctgcatgaa accagtcata 5820
ctaacagatg gtgaagagcg ggtgattctg gcaggaccta tgccagtgac ccactctagt 5880
gcagcacaaa gaagagggag aataggaaga aatccaaaaa atgagaatga ccagtacata 5940
tacatggggg aacctctgga aaatgatgaa gactgtgcac actggaaaga agctaaaatg 6000
ctcctagata acatcaacac gccagaagga atcattccta gcatgttcga accagagcgt 6060
gaaaaggtgg atgccattga tggcgaatac cgcttgagag gagaagcaag gaaaaccttt 6120
gtagacttaa tgagaagagg agacctacca gtctggttgg cctacagagt ggcagctgaa 6180
ggcatcaact acgcagacag aaggtggtgt tttgatggag tcaagaacaa ccaaatccta 6240
gaagaaaacg tggaagttga aatctggaca aaagaagggg aaaggaagaa attgaaaccc 6300
agatggttgg atgctaggat ctattctgac ccactggcgc taaaagaatt taaggaattt 6360
gcagccggaa gaaagtctct gaccctgaac ctaatcacag aaatgggtag gctcccaacc 6420
ttcatgactc agaaggcaag agacgcactg gacaacttag cagtgctgca cacggctgag 6480
gcaggtggaa gggcgtacaa ccatgctctc agtgaactgc cggagaccct ggagacattg 6540
cttttactga cacttctggc tacagtcacg ggagggatct ttttattctt gatgagcgca 6600
aggggcatag ggaagatgac cctgggaatg tgctgcataa tcacggctag catcctccta 6660
tggtacgcac aaatacagcc acactggata gcagcttcaa taatactgga gttttttctc 6720
atagttttgc ttattccaga acctgaaaaa cagagaacac cccaagacaa ccaactgacc 6780
tacgttgtca tagccatcct cacagtggtg gccgcaacca tggcaaacga gatgggtttc 6840
ctagaaaaaa cgaagaaaga tctcggattg ggaagcattg caacccagca acccgagagc 6900
aacatcctgg acatagatct acgtcctgca tcagcatgga cgctgtatgc cgtggccaca 6960
acatttgtta caccaatgtt gagacatagc attgaaaatt cctcagtgaa tgtgtcccta 7020
acagctatag ccaaccaagc cacagtgtta atgggtctcg ggaaaggatg gccattgtca 7080
aagatggaca tcggagttcc ccttctcgcc attggatgct actcacaagt caaccccata 7140
actctcacag cagctctttt cttattggta gcacattatg ccatcatagg gccaggactc 7200
caagcaaaag caaccagaga agctcagaaa agagcagcgg cgggcatcat gaaaaaccca 7260
actgtcgatg gaataacagt gattgaccta gatccaatac cttatgatcc aaagtttgaa 7320
aagcagttgg gacaagtaat gctcctagtc ctctgcgtga ctcaagtatt gatgatgagg 7380
actacatggg ctctgtgtga ggctttaacc ttagctaccg ggcccatctc cacattgtgg 7440
gaaggaaatc cagggaggtt ttggaacact accattgcgg tgtcaatggc taacattttt 7500
agagggagtt acttggccgg agctggactt ctcttttcta ttatgaagaa cacaaccaac 7560
acaagaaggg gaactggcaa cataggagag acgcttggag agaaatggaa aagccgattg 7620

aacgcattgg gaaaaagtga attccagatc tacaagaaaa gtggaatcca ggaagtggat 7680
agaaccttag caaaagaagg cattaaaaga ggagaaacgg accatcacgc tgtgtcgcga 7740
ggctcagcaa aactgagatg gttcgttgag agaaacatgg tcacaccaga agggaaagta 7800
gtggacctcg gttgtggcag aggaggctgg tcatactatt gtggaggact aaagaatgta 7860
agagaagtca aaggcctaac aaaaggagga ccaggacacg aagaacccat ccccatgtca 7920
acatatgggt ggaatctagt gcgtcttcaa agtggagttg acgttttctt catcccgcca 7980
gaaaagtgtg acacattatt gtgtgacata ggggagtcat caccaaatcc cacagtggaa 8040
gcaggacgaa cactcagagt ccttaactta gtagaaaatt ggttgaacaa caacactcaa 8100
ttttgcataa aggttctcaa cccatatatg ccctcagtca tagaaaaaat ggaagcacta 8160
caaaggaaat atggaggagc cttagtgagg aatccactct cacgaaactc cacacatgag 8220
atgtactggg tatccaatgc ttccgggaac atagtgtcat cagtgaacat gatttcaagg 8280
atgttgatca acagatttac aatgagatac aagaaagcca cttacgagcc ggatgttgac 8340
ctcggaagcg gaacccgtaa catcgggatt gaaagtgaga taccaaacct agatataatt 8400
gggaaaagaa tagaaaaaat aaagcaagag catgaaacat catggcacta tgaccaagac 8460
cacccataca aaacgtgggc ataccatggt agctatgaaa caaaacagac tggatcagca 8520
tcatccatgg tcaacggagt ggtcaggctg ctgacaaaac cttgggacgt tgtccccatg 8580
gtgacacaga tggcaatgac agacacgact ccatttggac aacagcgcgt ttttaaagag 8640
aaagtggaca cgagaaccca agaaccgaaa gaaggcacga agaaactaat gaaaataaca 8700
gcagagtggc tttggaaaga attagggaag aaaaagacac ccaggatgtg caccagagaa 8760
gaattcacaa gaaaggtgag aagcaatgca gccttggggg ccatattcac tgatgagaac 8820
aagtggaagt cggcacgtga ggctgttgaa gatagtaggt tttgggagct ggttgacaag 8880
gaaaggaatc tccatcttga aggaaagtgt gaaacatgtg tgtacaacat gatgggaaaa 8940
agagagaaga agctagggga attcggcaag gcaaaaggca gcagagccat atggtacatg 9000
tggcttggag cacgcttctt agagtttgaa gccctaggat tcttaaatga agatcactgg 9060
ttctccagag agaactccct gagtggagtg gaaggagaag ggctgcacaa gctaggttac 9120
attctaagag acgtgagcaa gaaagaggga ggagcaatgt atgccgatga caccgcagga 9180
tgggatacaa aaatcacact agaagaccta aaaaatgaag agatggtaac aaaccacatg 9240
gaaggagaac acaagaaact agccgaggcc attttcaaac taacgtacca aaacaaggtg 9300
gtgcgtgtgc aaagaccaac accaagaggc acagtaatgg acatcatatc gagaagagac 9360
caaagaggta gtggacaagt tggcacctat ggactcaata ctttcaccaa tatggaagcc 9420
caactaatca gacagatgga gggagaagga gtctttaaaa gcattcagca cctaacaatc 9480
acagaagaaa tcgctgtgca aaactggtta gcaagagtgg ggcgcgaaag gttatcaaga 9540
atggccatca gtggagatga ttgtgttgtg aaacctttag atgacaggtt cgcaagcgct 9600

ttaacagctc taaatgacat gggaaagatt aggaaagaca tacaacaatg ggaaccttca 9660
agaggatgga atgattggac acaagtgccc ttctgttcac accatttcca tgagttaatc 9720
atgaaagacg gtcgcgtact cgttgttcca tgtagaaacc aagatgaact gattggcaga 9780
gcccgaatct cccaaggagc agggtggtct ttgcgggaga cggcctgttt ggggaagtct 9840
tacgcccaaa tgtggagctt gatgtacttc cacagacgcg acctcaggct ggcggcaaat 9900
gctatttgct cggcagtacc atcacattgg gttccaacaa gtcgaacaac ctggtccata 9960
catgctaaac atgaatggat gacaacggaa gacatgctga cagtctggaa cagggtgtgg 10020
attcaagaaa acccatggat ggaagacaaa actccagtgg aaacatggga ggaaatccca 10080
tacttgggga aaagagaaga ccaatggtgc ggctcattga ttgggttaac aagcagggcc 10140
acctgggcaa agaacatcca agcagcaata aatcaagtta gatcccttat aggcaatgaa 10200
gaatacacag attacatgcc atccatgaaa agattcagaa gagaagagga agaagcagga 10260
gttctgtggt agaaagcaaa actaacatga aacaaggcta gaagtcaggt cggattaagc 10320
catagtacgg aaaaaactat gctacctgtg agccccgtcc aaggacgtta aaagaagtca 10380
ggccatcata aatgccatag cttgagtaaa ctatgcagcc tgtagctcca cctgagaagg 10440
tgtaaaaaat ccgggaggcc acaaaccatg gaagctgtac gcatggcgta gtggactagc 10500
ggttagggga gacccctccc ttacaaatcg cagcaacaat gggggcccaa ggcgagatga 10560
agctgtagtc tcgctggaag gactagaggt tagaggagac ccccccgaaa caaaaaacag 10620
catattgacg ctgggaaaga ccagagatcc tgctgtctcc tcagcatcat tccaggcaca 10680
gaacgccaga aaatggaatg gtgctgttga atcaacaggt tct 10723

**Revendications**

1. Kit d'immunisation contre le virus de la dengue comprenant un boitier contenant au moins les virus vaccinaux de la dengue d'un premier et d'un deuxième sérotype,

   (a) sous la forme de 2 compositions monovalentes contenues dans 2 récipients séparés, ou
   (b) sous la forme d'une composition bivalente contenue dans 1 seul récipient.

2. Kit selon la revendication 1 comprenant au moins :

   (a) un récipient contenant un vaccin bivalent comprenant un ChimeriVax™ DEN-1 et un ChimeriVax™ DEN-3, ou
   (b) un récipient contenant un vaccin bivalent comprenant un ChimeriVax™ DEN-2 et un ChimeriVax™ DEN-4, ou
   (c) un récipient contenant un vaccin bivalent comprenant un ChimeriVax™ DEN-1 et un ChimeriVax™ DEN-2, ou
   (d) un récipient contenant un vaccin bivalent comprenant un ChimeriVax™ DEN-3 et un ChimeriVax™ DEN-4.

3. Vaccin bivalent comprenant une quantité immunoefficace les virus vaccinaux de la dengue d'un premier et d'un deuxième sérotype et un excipient pharmaceutiquement acceptable.

4. Vaccin bivalent selon la revendication 3 comprenant les virus vaccinaux sélectionnés dans le groupe consistant en : ChimeriVax™ DEN-1 et ChimeriVax™ DEN-3 ; ou ChimeriVax™ DEN-2 et ChimeriVax™ DEN-4 ; ou ChimeriVax™ DEN-1 et un ChimeriVax™ DEN-2 ; ou ChimeriVax™ DEN-3 et ChimeriVax™ DEN-4.

Europäisches Patentamt
European Patent Office
Office européen des brevets

# RAPPORT DE RECHERCHE EUROPEENNE

Numéro de la demande
EP 11 19 2672

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X<br><br>Y | EP 1 159 968 A (MAHIDOL UNIVERSITY [TH])<br>5 décembre 2001 (2001-12-05)<br>* page 3, ligne 43 - ligne 55 *<br>* page 4, ligne 4 - ligne 6; exemples 5,7 *<br>----- | 1,3<br><br>2,4 | INV.<br>A61K39/12<br>A61P31/14 |
| X | US 2004/120964 A1 (MIKSZTA JOHN A [US] ET AL) 24 juin 2004 (2004-06-24)<br>* page 3, alinéa 0024 *<br>* page 4, alinéa 0036 *<br>* page 5, alinéa 0039 *<br>* page 6, alinéa 0046 *<br>* page 7, alinéa 0057 *<br>* page 8, alinéa 0061 *<br>* page 10, alinéa 0079 *<br>* page 14, alinéa 0118 - alinéa 0119 *<br>----- | 1-4 | |
| X<br><br><br>Y | WO 03/103571 A2 (ACAMBIS INC [US]; MONATH THOMAS P [US]; GUIRAKHOO FARSHAD [US]; ARROYO) 18 décembre 2003 (2003-12-18)<br>* page 7, ligne 8 - ligne 23 *<br>* page 10, ligne 11 - ligne 16 *<br>----- | 1<br><br><br>2-4 | DOMAINES TECHNIQUES RECHERCHES (IPC)<br>A61K |
| X<br><br><br>Y | WO 03/101397 A (ACAMBIS INC [US]; GUIRAKHOO FARSHAD [US])<br>11 décembre 2003 (2003-12-11)<br>* abrégé; exemples 1-6 *<br>* page 7, ligne 27 - ligne 30; revendications 1-18 *<br>-----<br>-/-- | 1<br><br><br>2-4 | |

Le présent rapport a été établi pour toutes les revendications

| Lieu de la recherche | Date d'achèvement de la recherche | Examinateur |
|---|---|---|
| Munich | 17 octobre 2012 | Loubradou, Gabriel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

4

EPO FORM 1503 03.82 (P04C02)

Europäisches Patentamt
European Patent Office
Office européen des brevets

# RAPPORT DE RECHERCHE EUROPEENNE

Numéro de la demande
EP 11 19 2672

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y,D | ZHOU ET AL: "Sculpting the immunological response to dengue fever by polytopic vaccination", VACCINE, BUTTERWORTH SCIENTIFIC. GUILDFORD, GB, vol. 24, no. 14, 24 mars 2006 (2006-03-24), pages 2451-2459, XP005309251, ISSN: 0264-410X<br>* abrégé *<br>* page 2452, colonne de gauche, alinéa 1 - colonne de droite, alinéa 1 *<br>* page 2454, colonne de gauche, dernier alinéa - colonne de droite, dernier alinéa *<br>* page 2455, colonne de gauche, dernier alinéa - colonne de droite, alinéa 3 *<br>----- | 2-4 | |
| X,P | WO 2007/021672 A2 (ACAMBIS INC [US]; SANOFI PASTEUR SA [FR]; MONATH THOMAS P [US]; GUIRAK) 22 février 2007 (2007-02-22)<br>* page 16, ligne 23 - ligne 31 *<br>* page 17, ligne 25 - ligne 27 *<br>* page 18, ligne 4 - ligne 9 *<br>----- | 1-4 | DOMAINES TECHNIQUES RECHERCHES (IPC) |
| E | WO 2008/007021 A1 (SANOFI PASTEUR [FR]) 17 janvier 2008 (2008-01-17)<br>* page 13, ligne 27 - page 14, ligne 18 *<br>* page 16, ligne 7 - ligne 16 *<br>* page 17, ligne 31 - page 18, ligne 6 *<br>----- | 1-4 | |

Le présent rapport a été établi pour toutes les revendications

| Lieu de la recherche | Date d'achèvement de la recherche | Examinateur |
|---|---|---|
| Munich | 17 octobre 2012 | Loubradou, Gabriel |

4

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 11 19 2672

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-10-2012

| Document brevet cité au rapport de recherche | Date de publication | Membre(s) de la famille de brevet(s) | Date de publication |
|---|---|---|---|
| EP 1159968 A | 05-12-2001 | AT 412738 T | 15-11-2008 |
| | | DK 1159968 T3 | 23-02-2009 |
| | | EP 1159968 A1 | 05-12-2001 |
| | | ES 2315221 T3 | 01-04-2009 |
| | | PT 1159968 E | 20-11-2008 |
| US 2004120964 A1 | 24-06-2004 | US 2004120964 A1 | 24-06-2004 |
| | | WO 2005067968 A1 | 28-07-2005 |
| WO 03103571 A2 | 18-12-2003 | AU 2003267937 A1 | 22-12-2003 |
| | | BR 0306905 A | 12-04-2005 |
| | | CA 2473321 A1 | 18-12-2003 |
| | | EP 1471873 A2 | 03-11-2004 |
| | | EP 2295023 A1 | 16-03-2011 |
| | | JP 2005519639 A | 07-07-2005 |
| | | JP 2010268804 A | 02-12-2010 |
| | | KR 20110013494 A | 09-02-2011 |
| | | MX PA04006870 A | 16-05-2005 |
| | | NZ 534159 A | 30-03-2007 |
| | | US 2008274142 A1 | 06-11-2008 |
| | | US 2009191240 A1 | 30-07-2009 |
| | | WO 03103571 A2 | 18-12-2003 |
| WO 03101397 A | 11-12-2003 | AU 2003239932 A1 | 19-12-2003 |
| | | US 2004259224 A1 | 23-12-2004 |
| | | US 2010158938 A1 | 24-06-2010 |
| | | WO 03101397 A2 | 11-12-2003 |
| WO 2007021672 A2 | 22-02-2007 | AR 055603 A1 | 29-08-2007 |
| | | AU 2006280144 A1 | 22-02-2007 |
| | | BR PI0614265 A2 | 22-03-2011 |
| | | CA 2618783 A1 | 22-02-2007 |
| | | EP 1924280 A2 | 28-05-2008 |
| | | JP 2009504654 A | 05-02-2009 |
| | | US 2008193477 A1 | 14-08-2008 |
| | | WO 2007021672 A2 | 22-02-2007 |
| WO 2008007021 A1 | 17-01-2008 | AR 061887 A1 | 01-10-2008 |
| | | AU 2007274100 A1 | 17-01-2008 |
| | | BR PI0712878 A2 | 04-09-2012 |
| | | CA 2656349 A1 | 17-01-2008 |
| | | CN 101489585 A | 22-07-2009 |
| | | EP 2043680 A1 | 08-04-2009 |
| | | FR 2903605 A1 | 18-01-2008 |
| | | JP 2009542783 A | 03-12-2009 |
| | | KR 20090027759 A | 17-03-2009 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 11 19 2672

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-10-2012

| Document brevet cité au rapport de recherche | Date de publication | Membre(s) de la famille de brevet(s) | Date de publication |
|---|---|---|---|
| | | TW 200813228 A | 16-03-2008 |
| | | US 2008014219 A1 | 17-01-2008 |
| | | US 2010221285 A1 | 02-09-2010 |
| | | WO 2008007021 A1 | 17-01-2008 |
| | | ZA 200900155 A | 31-03-2010 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0266621 A
- WO 0057904 A
- WO 0057908 A
- WO 0057909 A
- WO 0057910 A
- WO 020950075 A
- WO 02102828 A
- WO 9837911 A
- WO 9640933 A
- WO 0160847 A
- EP 1159968 A
- WO 03101397 A


### Littérature non-brevet citée dans la description


- **GÜBLER et al.** Epidemiology of arthropod-borne viral disease. CRC Press, 1988, 223-60
- **KAUTNER et al.** *J. of Pediatrics,* 1997, vol. 131, 516-524
- **RIGAU-PÉREZ et al.** *Lancet,* 1998, vol. 352, 971-977
- **VAUGHN et al.** *J Infect Dis,* 1997, vol. 176, 322-30
- **VAUGHN et al.** *J. Infect. Dis.,* 1997, vol. 176, 322-30
- WHO technical Guide, 1986. Dengue haemorrhagic fever: diagnosis, treatment and control. 1-2
- **GUBLER.** *TRENDS in Microbiology,* 2002, vol. 10, 100-103
- **MONATH.** *Proc. Natl. Acad. Sci.,* 1994, vol. 91, 2395-2400
- **SHIRTCLIFFE et al.** *J. Roy. Coll. Phys. Lond.,* 1998, vol. 32, 235-237
- **DEFRAITES et al.** *MMWR,* 1994, vol. 43, 845-848
- **WU et al.** *Nature Med.,* 2000, vol. 7, 816-820
- **SABIN.** *Am. J. Trop. Med. Hyg.,* 1952, vol. 1, 30-50
- **VAUGHN et al.** *J. Inf. Dis.,* 2000, vol. 181, 2-9
- **ROTHMAN ; ENNIS.** *Virology,* 1999, vol. 257, 1-6
- **HALSTEAD et al.** *Am. J. Trop. Med. Hyg.,* 1973, vol. 22, 375-381
- **GUBLER D.J.** *Clin. Microbiol. Rev.,* 1998, vol. 11 (3), 480-96
- **ROTHMAN A. L. et al.** *Vaccine,* 2001, vol. 19, 4694-9
- **ZHOU H ; DEEM MW.** *Vaccine,* 24 Mars 2006, vol. 24 (14), 2451-9
- **THEILER M ; SMITH HH.** *J Exp. Med,* 1937, vol. 65, 767-786
- **RICE et al.** *Science,* 1985, vol. 229, 726-733